# EUROPÄISCHE PATENTSCHRIFT

(11) **EP 0 793 657 B1**
(45) Veröffentlichungstag und Bekanntmachung des Hinweises auf die Patenterteilung: **03.05.2000**
(21) Anmeldenummer: 95938436.3
(22) Anmeldetag: 08.11.1995
(51) Int. Cl.: C07D 249/12, C07D 401/06, C07D 405/06, A01N 43/653

(54) **MIKROBIZIDE TRIAZOLYL-DERIVATE**
MICROBICIDAL TRIAZOLYL DERIVATIVES
DERIVES TRIAZOLYLE MICROBICIDES

(30) Priorität: 21.11.1994 DE 4441354; 24.07.1995 DE 19526918; 31.07.1995 DE 19528046
(43) Veröffentlichungstag der Anmeldung: 10.09.1997
(60) Teilanmeldung: 99106021.1
(73) Patentinhaber: BAYER AG, 51368 Leverkusen (DE)
(72) Erfinder: JAUTELAT, Manfred, D-51399 Burscheid (DE); TIEMANN, Ralf, D-51375 Leverkusen (DE); DUTZMANN, Stefan, D-40721 Hilden (DE); HÄNSSLER, Gerd, D-51381 Leverkusen (DE); STENZEL, Klaus, D-40595 Düsseldorf (DE)
(86) Internationale Anmeldenummer: EP9504392
(87) Internationale Veröffentlichungsnummer: WO9616048

(56) Entgegenhaltungen:
- EP-A- 0 251 086
- EP-A- 0 297 345
- EP-A- 0 461 502
- EP-A- 0 564 810

## Beschreibung

Die vorliegende Erfindung betrifft neue Triazolyl-Derivate, mehrere Verfahren zu deren Herstellung und deren Verwendung als Mikrobizide.

Es ist bereits bekannt geworden, daß zahlreiche Hydroxyethyl-azolyl-Derivate fungizide Eigenschaften besitzen (vgl. EP-A 0 015 756, EP-A 0 040 345, EP-A 0 052 424, EP-A 0 061 835 und EP-A 0 297 345). Die Einsetzbarkeit dieser Stoffe ist jedoch in manchen Fällen nicht immer befriedigend.

Weiterhin sind bereits fungizid wirksame Triazolyl - Derivate bekannt, in denen der Triazol- Rest eine Mercapto - Gruppe trägt und das Carbinol-Kohlenstoffatom mit einer Alkenyl- oder Aralkenyl- Gruppe verbunden ist ( vgl. EP - A 0 251 086 ). Verbindungen dieses Typs, die am zentralen Kohlenstoffatom keine Alkenyl- oder Aralkenyl- Gruppe enthalten, werden jedoch nicht offenbart.

Es wurden nun neue Triazolyl-Derivate der Formel 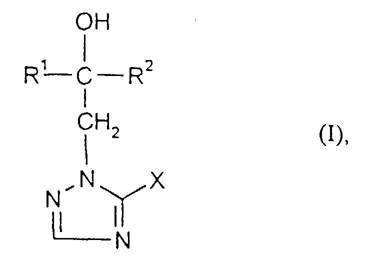 in welcher
- R¹: für geradkettiges oder verzweigtes Alkyl mit 1 bis 6 Kohlenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
- R¹: für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
- R¹: für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
- R¹: für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
- R¹: für Aryl mit 6 bis 10 Kohlenstoffatomen steht, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
- R¹: für einen gegebenenfalls benzanellierten fünf- oder sechsgliedrigen heteroaromatischen Rest mit 1 bis 3 Heteroatomen steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halo-, genatomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano,
- R²: für geradkettiges oder verzweigtes Alkyl mit 1 bis 6 Kohlenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
- R²: für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
- R²: für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
- R²: für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder vefschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
- R²: für Aryl mit 6 bis 10 Kohlenstoffatomen steht, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
- R²: für einen gegebenenfalls benzanellierten fünf- oder sechsgliedrigen heteroaromatischen Rest mit 1 bis 3 Heteroatomen steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano,
und
- X: für die Gruppierungen -SH, -SR³, -SO-R³, -SO₂-R³ oder -SO₃H steht, worin
- R³: für geradkettiges oder verzweigtes Alkyl mit 1 bis 6 Kohlenstoffatomen steht, wobei jeder dieser Reste einfach bis dreifach durch Fluor und/oder Chlor substituiert sein kann,
oder
- R³: für geradkettiges oder verzweigtes Alkenyl mit 2 bis 6 Kohlenstoffatomen steht, wobei jeder dieser Reste einfach bis dreifach durch Fluor und/oder Chlor substituiert sein kann,
oder
- R³: für Phenylalkyl mit 1 bis 4 Kohlenstoffatomen im geradkettigen oder verzweigten Alkylteil steht, wobei jeder dieser Reste im Phenylteil einfach bis dreifach, gleichartig oder verschieden substituiert sein kann durch Halogen, Alkyl mit 1 bis 4 Kohlenstoffatomen und/oder Halogenalkyl mit 1 bis 4 Kohlenstoffatomen und 1 bis 5 Halogenatomen,
oder
- R³: für Phenyl steht, das einfach bis dreifach, gleichartig oder verschieden substituiert sein kann durch Halogen, Alkyl mit 1 bis 4 Kohlenstoffatomen und/oder Halogenalkyl mit 1 bis 4 Kohlenstoffatomen und 1 bis 5 Halogenatomen,
sowie deren Säureadditions-Salze und Metallsalz-Komplexe gefunden.

Diejenigen erfindungsgemäßen Stoffe, in denen R¹ und R² verschieden sind, enthalten ein asymmetrisch substituiertes Kohlenstoffatom. Sie können daher in optischen Isomerenformen anfallen. Die vorliegende Erfindung betrifft sowohl die einzelnen Isomeren als auch deren Gemische.

Weiterhin wurde gefunden, daß man Triazolyl-Derivate der Formel (I) sowie deren Säureadditions-Salze und Metallsalz-Komplexe erhält, wenn man
a) Hydroxyethyl-triazole der Formel 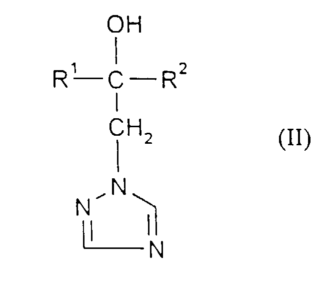 in welcher
   R¹ und R² die oben angegebenen Bedeutungen haben,
   entweder
   α) nacheinander mit starken Basen und Schwefel in Gegenwart eines Verdünnungsmittels umsetzt und dann mit Wasser, gegebenenfalls in Gegenwart einer Säure hydrolysiert,
      oder
   β) mit Schwefel in Gegenwart eines hoch siedenden Verdünnungsmittels umsetzt und dann gegebenenfalls mit Wasser sowie gegebenenfalls mit Säure behandelt,
   und gegebenenfalls die nach den Varianten (α) und (β) entstehenden Verbindungen der Formel 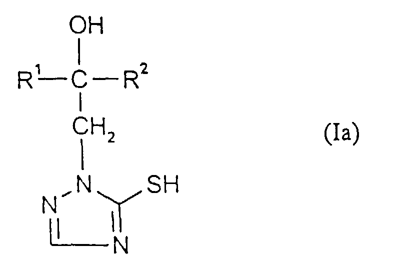 in welcher
   R¹ und R² die oben angegebenen Bedeutungen haben,
   mit Halogen-Verbindungen der Formel

   R⁴-Hal (III)

   in welcher
   - R⁴: für geradkettiges oder verzweigtes Alkyl mit 1 bis 6 Kohlenstoffatomen steht, wobei jeder dieser Reste einfach bis dreifach durch Fluor und/oder Chlor substituiert sein kann,
   oder
   für geradkettiges oder verzweigtes Alkenyl mit 2 bis 6 Kohlenstoffatomen steht, wobei jeder dieser Reste einfach bis dreifach durch Fluor und/oder Chlor substituiert sein kann,
   oder
   für Phenylalkyl mit 1 bis 4 Kohlenstoffatomen im geradkettigen oder verzweigten Alkylteil steht, wobei jeder dieser Reste im Phenylteil einfach bis dreifach, gleichartig oder verschieden substituiert sein kann durch Halogen, Alkyl mit 1 bis 4 Kohlenstoffatomen und/oder Halogenalkyl mit 1 bis 4 Kohlenstoffatomen und 1 bis 5 Halogenatomen,
   und
   - Hal: für Chlor, Brom oder Iod steht,
   in Gegenwart eines Säurebindemittels und in Gegenwart eines Verdünnungsmittels umsetzt und gegebenenfalls die dabei entstehenden Verbindungen der Formel 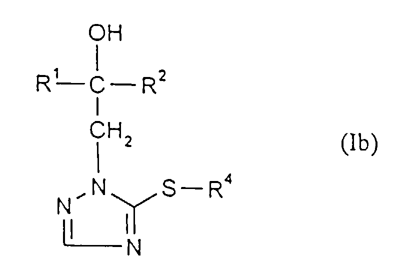 in welcher
   R¹, R² und R⁴ die oben angegebenen Bedeutungen haben,
   mit Oxidationsmitteln in Gegenwart eines Verdünnungsmittels umsetzt,
   oder
b) Hydroxyethyl-triazole der Formel 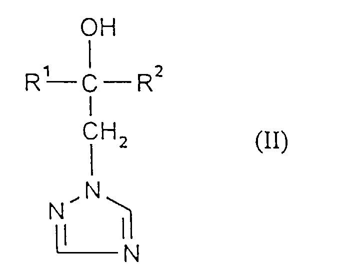 in welcher
   R¹ und R² die oben angegebenen Bedeutungen haben,
   nacheinander mit starken Basen und Diaryl-disulfiden der Formel

   R⁵-S-S-R⁵ (IV)

   in welcher
   - R⁵: für Phenyl steht, das einfach bis dreifach, gleichartig oder verschieden substituiert sein kann durch Halogen, Alkyl mit 1 bis 4 Kohlenstoffatomen und/oder Halogenalkyl mit 1 bis 4 Kohlenstoffatomen und 1 bis 5 Halogenatomen,
   in Gegenwart eines Verdünnungsmittels umsetzt und gegebenenfalls die dabei entstehenden Verbindungen der Formel 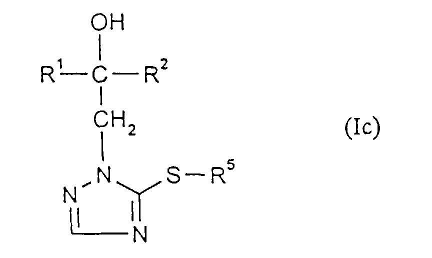 in welcher
   R¹, R² und R⁵ die oben angegebenen Bedeutungen haben,
   mit Oxidationsmitteln in Gegenwart eines Verdünnungsmittels umsetzt,
   oder
c) Triazolyl-Derivate der Formel 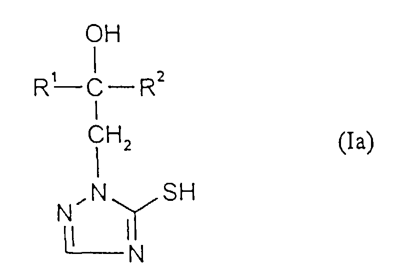 in welcher
   R¹ und R² die oben angegebenen Bedeutungen haben,
   mit Kaliumpermanganat in Gegenwart eines Verdünnungsmittels umsetzt,
und gegebenenfalls anschließend an die erhaltenen Verbindungen der Formel (I) eine Säure oder ein Metallsalz addiert.

Schließlich wurde gefunden, daß die neuen Triazolyl-Derivate der Formel (I) sowie deren Säureadditions-Salze und Metallsalz-Komplexe sehr gute mikrobizide Eigenschaften aufweisen und sich sowohl im Pflanzenschutz als auch im Materialschutz zur Bekämpfung unerwünschter Mikroorganismen, wie Fungi, verwenden lassen.

Überraschenderweise zeigen die erfindungsgemäßen Stoffe eine bessere mikrobizide Wirksamkeit, insbesondere fungizide Wirksamkeit, als die konstitutionell ähnlichsten Verbindungen gleicher Wirkungsrichtung.

Die erfindungsgemäßen Triazolyl-Derivate sind durch die Formel (I) allgemein definiert.
- R¹: steht bevorzugt für geradkettiges oder verzweigtes Alkyl mit 1 bis 4 Kohlenstoffatomen, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für Cycloalkyl mit 3 bis 6 Kohlenstoffatomen, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzimidazolyl, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximinoethyl, Nitro und/oder Cyano, Formyl, Dimethoxymethyl, Acetyl und/oder Propionyl.
- R²: steht bevorzugt für geradkettiges oder verzweigtes Alkyl mit 1 bis 4 Kohlenstoffatomen, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für Cycloalkyl mit 3 bis 6 Kohlenstoffatomen, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzimidazolyl, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximinoethyl, Nitro und/oder Cyano, Formyl, Dimethoxymethyl, Acetyl und/oder Propionyl.
- X: steht auch bevorzugt für die Gruppierungen -SH, -SR³, -SO-R³, -SO₂-R³ oder -SO₃H.
- R³: steht bevorzugt für geradkettiges oder verzweigtes Alkyl mit 1 bis 4 Kohlenstoffatomen, wobei jeder dieser Reste einfach bis dreifach durch Fluor und/oder Chlor substituiert sein kann,
oder
für geradkettiges oder verzweigtes Alkenyl mit 2 bis 5 Kohlenstoffatomen, wobei jeder dieser Reste einfach bis dreifach, gleichartig oder verschieden durch Fluor und/oder Chlor substituiert sein kann,
oder
für Phenylalkyl mit 1 bis 3 Kohlenstoffatomen im geradkettigen oder verzweigten Alkylteil, wobei jeder dieser Reste im Phenylteil einfach bis dreifach, gleichartig oder verschieden substituiert sein kann durch Fluor, Chlor, Brom, Methyl, Ethyl, tert.-Butyl, Trichlormethyl und/oder Trifluormethyl,
oder
für Phenyl, das einfach bis dreifach, gleichartig oder verschieden substituiert sein kann durch Fluor, Chlor, Brom, Methyl, Ethyl, tert.-Butyl, Trichlormethyl.
- R¹: steht besonders bevorzugt für n-Propyl, Isopropyl, n-Butyl, i-Butyl, sek.-Butyl oder tert.-Butyl, wobei diese Reste einfach bis vierfach, gleichartig oder verschieden substituiert sein können durch Fluor, Chlor, Brom, Methoxy, Ethoxy, Propoxy, Isopropoxy, Methoximino, Ethoximino, Cyclopropyl, Cyclobutyl, Cyclopentyl und/oder Cyclohexyl,
oder
für 1-Methyl-cyclohexyl, Cyclohexyl, 1-Chlor-cyclopropyl, 1 -Fluor-cyclopropyl, 1-Methyl-cyclopropyl, 1-Cyano-Cyclopropyl, Cyclopropyl, 1-Methyl-cyclopentyl oder 1-Ethyl-cyclopentyl,
oder
für Phenylalkyl mit 1 oder 2 Kohlenstoffatomen im geradkettigen oder verzweigten Alkylteil, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzimidazolyl, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximinoethyl, Nitro und/oder Cyano, Formyl, Dimethoxymethyl, Acetyl und/oder Propionyl.
- R²: steht besonders bevorzugt für n-Propyl, Isopropyl, n-Butyl, i-Butyl, sek.-Butyl oder tert.-Butyl, wobei diese Reste einfach bis vierfach, gleichartig oder verschieden substituiert sein können durch Fluor, Chlor, Brom, Methoxy, Ethoxy, Propoxy, Isopropoxy, Methoximino, Ethoximino, Cyclopropyl, Cyclobutyl, Cyclopentyl und/oder Cyclohexyl,
oder
für 1-Methyl-cyclohexyl, Cyclohexyl, 1-Chlor-cyclopropyl, 1-Fluor-cyclopropyl, 1-Methyl-cyclopropyl, 1-Cyano-cyclopropyl, Cyclopropyl, 1-Methyl-cyclopentyl oder 1-Ethyl-cyclopentyl,
oder
für Phenylalkyl mit 1 oder 2 Kohlenstoffatomen im geradkettigen oder verzweigten Alkylteil, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzimidazolyl, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximinoethyl, Nitro und/oder Cyano, Formyl, Dimethoxymethyl, Acetyl und/oder Propionyl.
- X: steht auch besonders bevorzugt für die Gruppierungen -SH, -SR³, -SO-R³, -SO₂-R³ oder -SO₃H.
- R³: steht besonders bevorzugt für Methyl, Ethyl oder Propyl, wobei jeder dieser Reste einfach bis dreifach, gleichartig oder verschieden durch Fluor und/oder Chlor substituiert sein kann,
oder
für Allyl, But-2-en-yl oder But-3-enyl, wobei jeder dieser Reste einfach bis dreifach, gleichartig oder verschieden substituiert sein kann durch Fluor und/oder Chlor,
oder
für Phenylalkyl mit 1 oder 2 Kohlenstoffatomen im geradkettigen oder verzweigten Alkylteil, wobei jeder dieser Reste im Phenylteil einfach oder zweifach substituiert sein kann durch Fluor, Chlor, Brom, Methyl, Ethyl, Tert.-Butyl, Trichlormethyl und/oder Trifluormethyl,
oder
für Phenyl, das einfach oder zweifach substituiert sein kann durch Fluor, Chlor, Brom, Methyl, Ethyl, tert.-Butyl, Trichlormethyl und/oder Trifluormethyl.

Bevorzugte erfindungsgemäße Verbindungen sind auch Additionsprodukte aus Säuren und denjenigen Triazolyl-Derivaten der Formel (I), in denen R¹, R² und X diejenigen Bedeutungen haben, die für diese Substituenten als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B.

Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure, sowie Saccharin und Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Triazolyl-Derivaten der Formel (I), in denen R¹, R² und X diejenigen Bedeutungen haben, die für diese Substituenten als bevorzugt genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Die erfindungsgemäßen Triazolyl-Derivate der Formel (I), in denen X für eine -SH-Gruppe steht, können in der "Mercapto"-Form der Formel 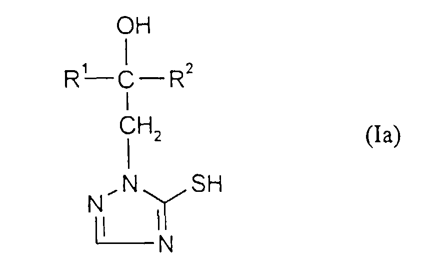 oder in der tautomeren "Thiono"-Form der Formel 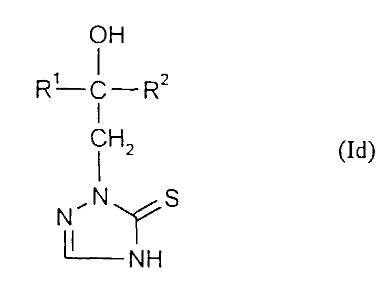 vorliegen. Der Einfachheit halber wird jeweils nur die "Mercapto"-Form aufgeführt.

Als Beispiele für erfindungsgemäße Stoffe seien die in der folgenden Tabelle aufgeführten Triazolyl-Derivate genannt.

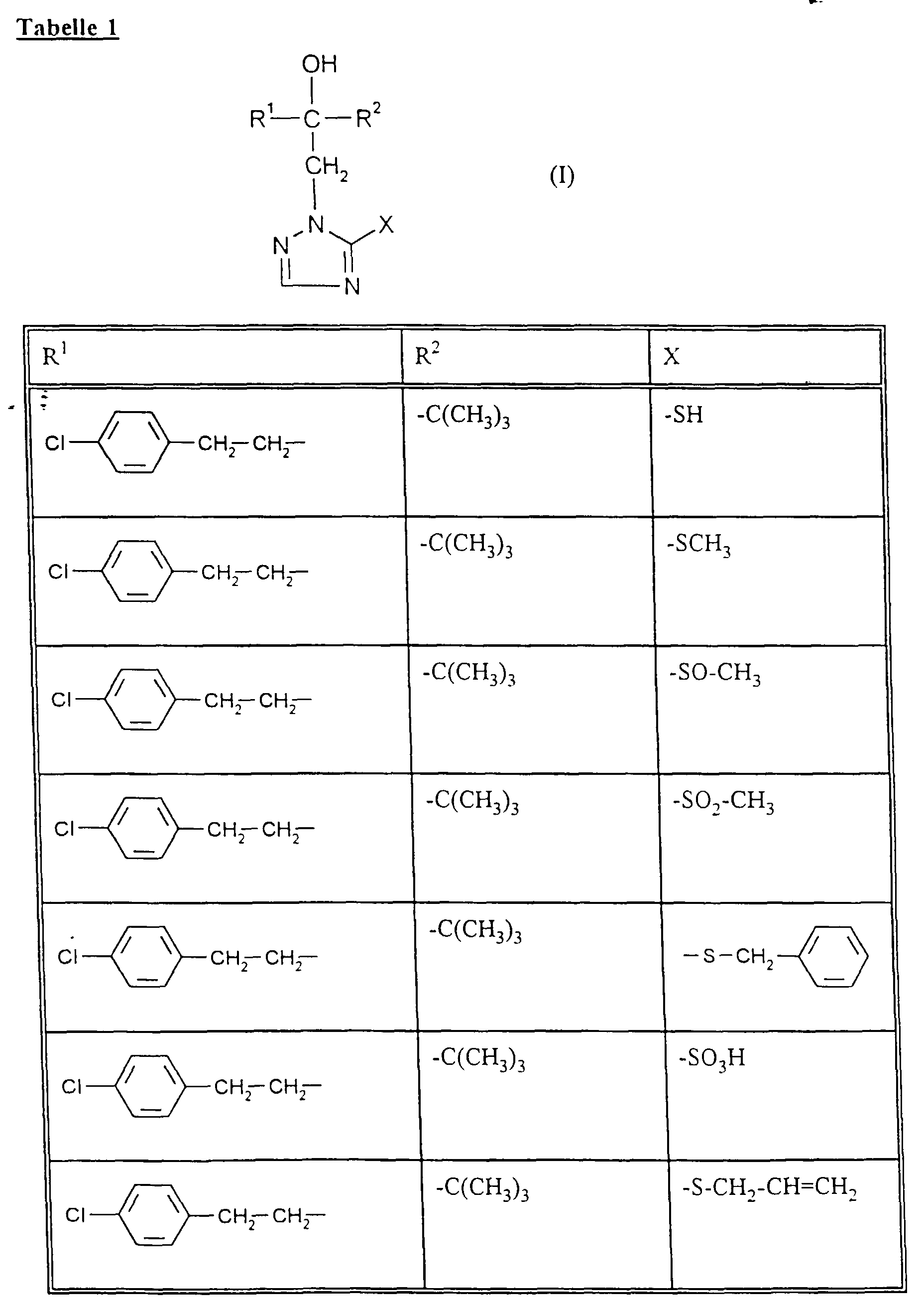

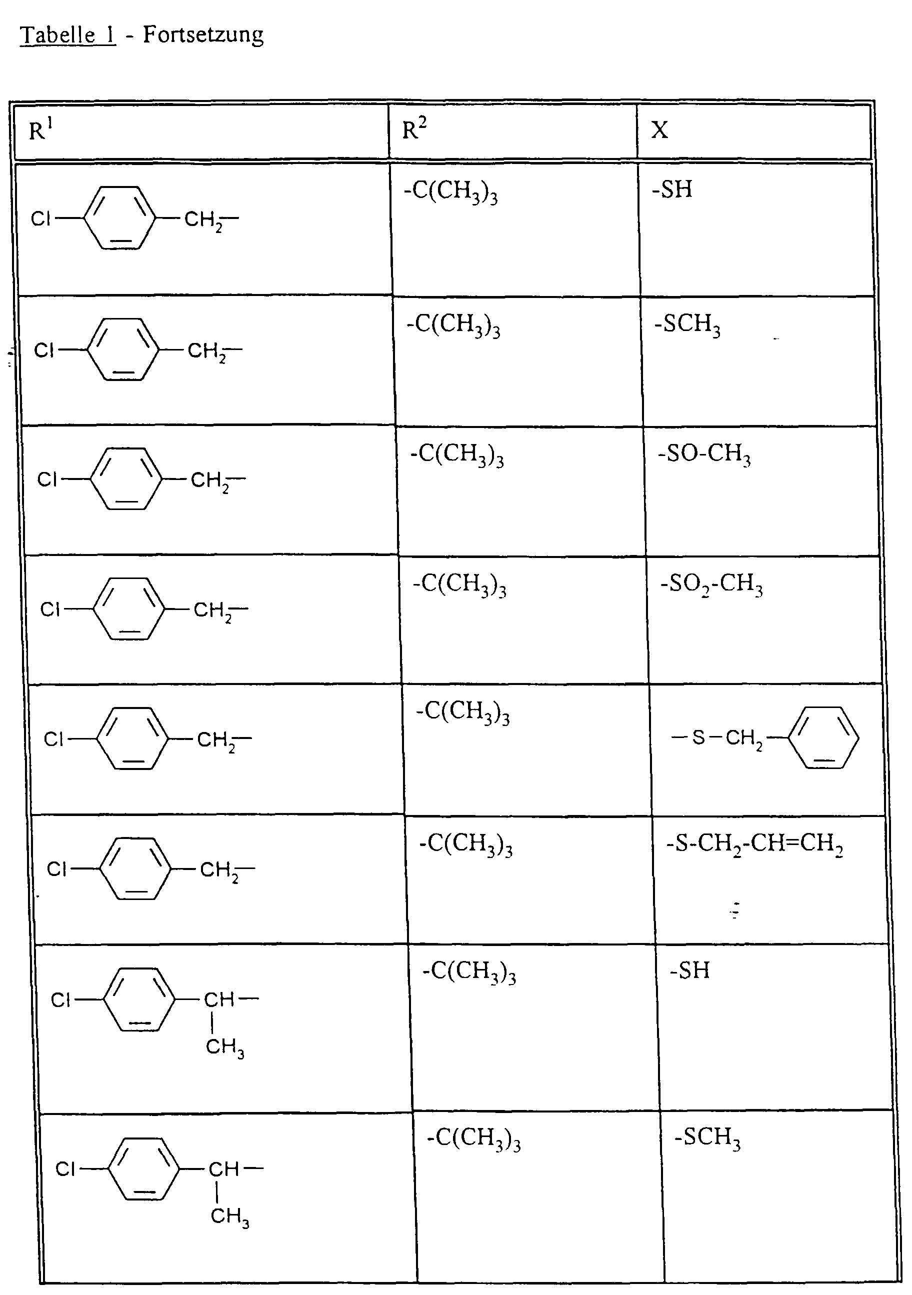

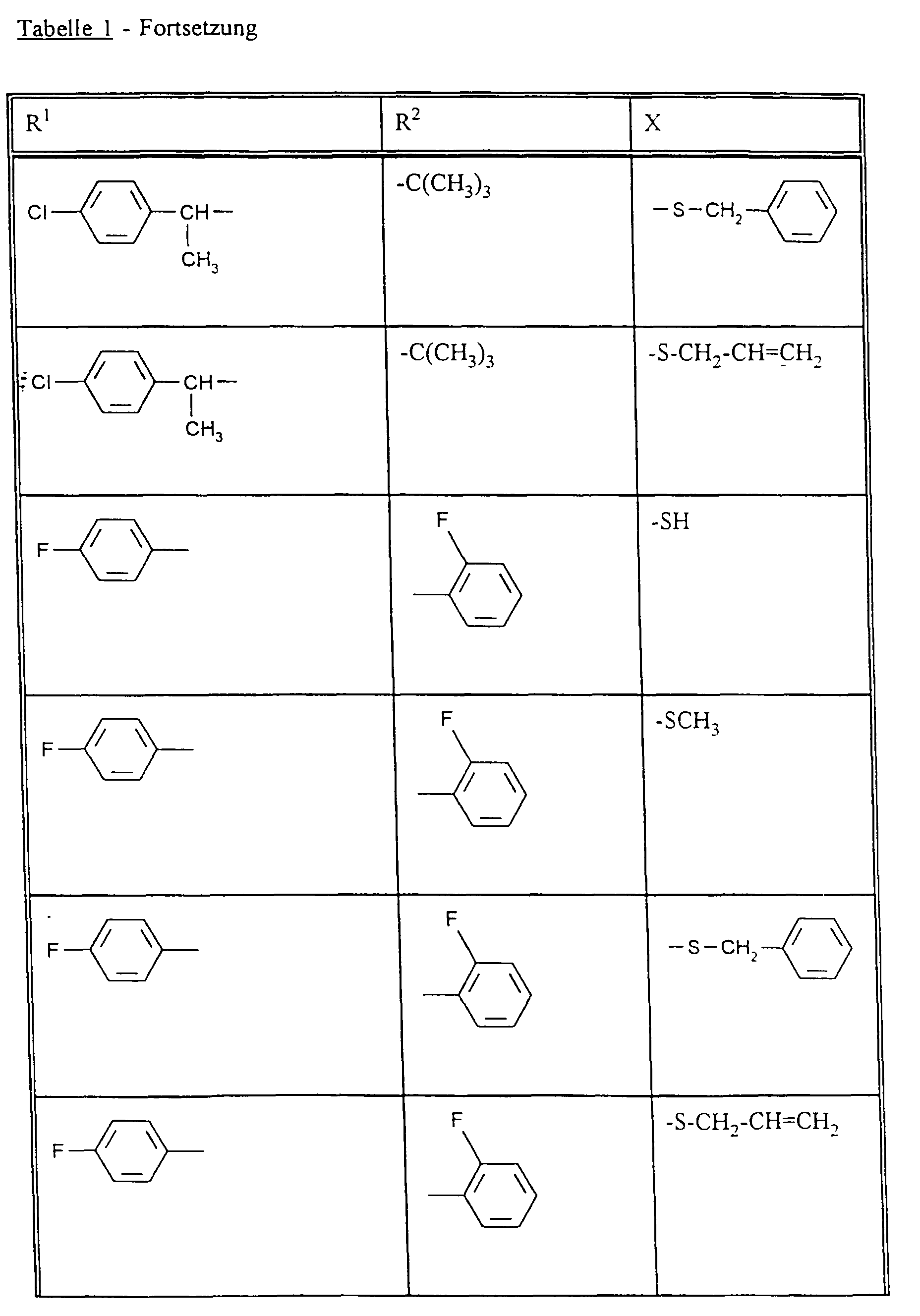

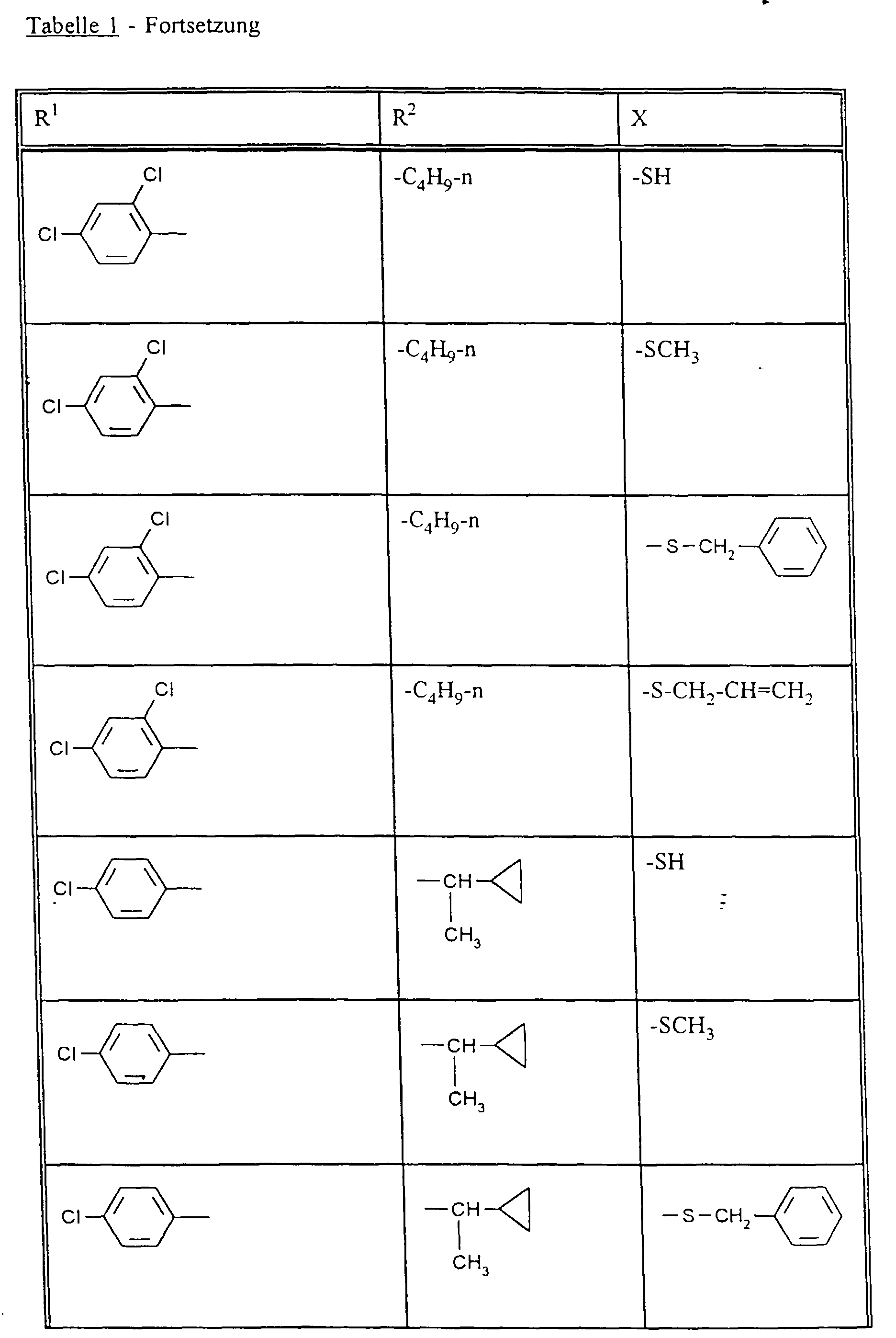

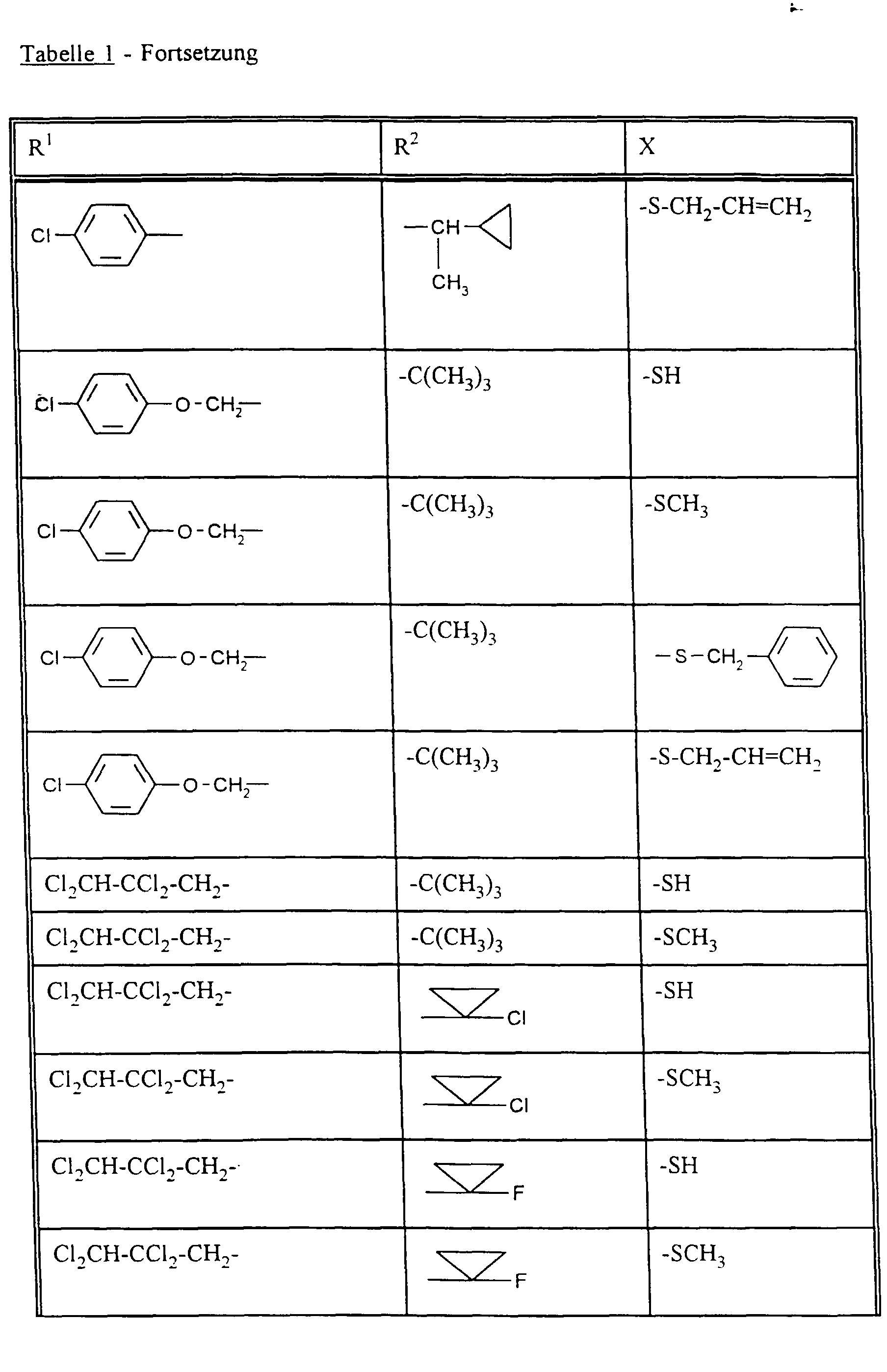

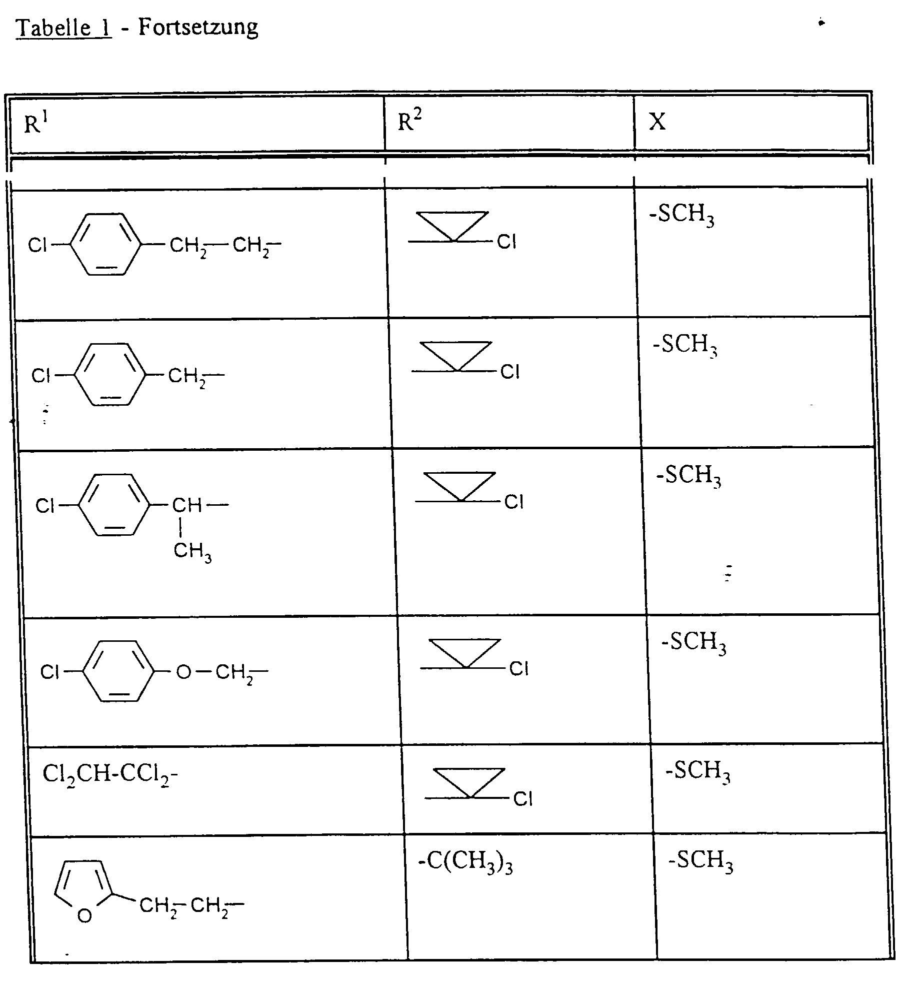

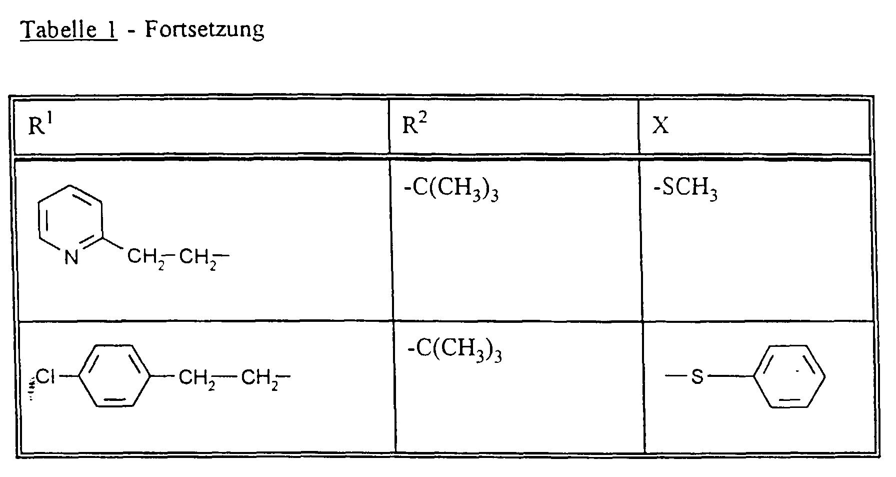

Verwendet man 2-(1-Chlor-cyclopropyl)-1-(2-chlorphenyl)-3-(1,2,4-triazol-1-yl)-propan-2-ol als Ausgangsstoff, n-Butyl-lithium als starke Base und Schwefel-Pulver als Reaktionskomponente, so kann der Verlauf der ersten Stufe des erfindungsgemäßen Verfahrens (a), Variante (α), durch das folgende Formelschema veranschaulicht werden:

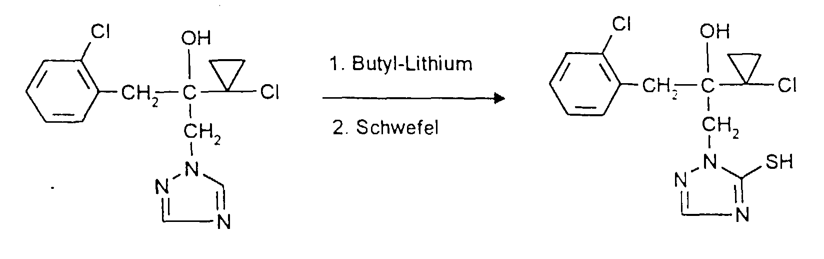

Verwendet man 2-(1-Chlor-cyclopropyl)-1-(2-chlor-phenyl)-3-(1,2,4-triazol-1-yl)-propan-2-ol als Ausgangsstoff, Schwefel-Pulver als Reaktionskomponente und N-Methyl-pyrrolidon als Verdünnungsmittel, so kann der Verlauf der ersten Stufe des erfindungsgemäßen Verfahrens (a), Variante (β) durch das folgende Formelschema veranschaulicht werden:

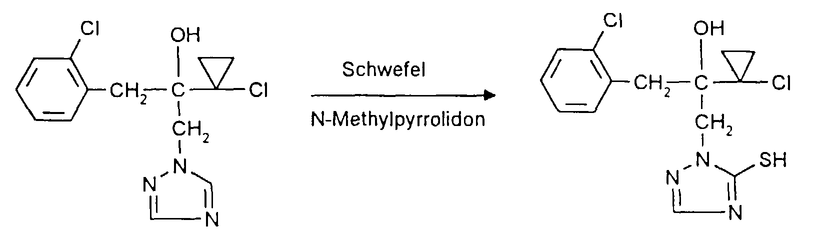

Verwendet man 2-(1-Chlor-cyclopropyl)-1-(2-chlorphenyl)-3-(5-mercapto-1,2,4-triazol-1-yl)-propan-2-ol als Ausgangsstoff und Methyliodid als Reaktionskomponente, so kann der Verlauf der zweiten Stufe des erfindungsgemäßen Verfahrens (a) durch das folgende Formelschema veranschaulicht werden:

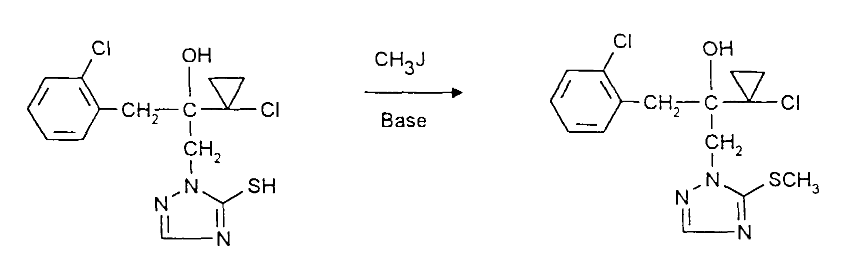

Verwendet man 2-(1-Chlor-cyclopropyl)-1-(2-chlorphenyl)-3-(5-methylthio-1,2,4-triazol-1-yl)-propan-2-ol als Ausgangsstoff und einen Überschuß an Wasserstoffperoxid als Oxidationsmittel, so kann der Verlauf der dritten Stufe des erfindungsgemäßen Verfahrens (a) durch das folgende Formelschema veranschaulicht werden:

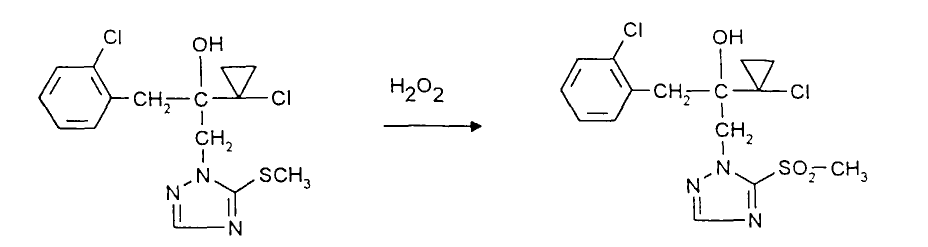

Verwendet man 2-(1-Chlor-cyclopropyl)-1-(2-chlorphenyl)-3-(1,2,4-triazol-1-yl)-propan-2-ol als Ausgangsstoff, n-Butyllithium als starke Base und Diphenyldisulfid als Reaktionskomponente, so kann der Verlauf der ersten Stufe des erfindungsgemäßen Verfahrens (b) durch das folgende Formelschema veranschaulicht werden:

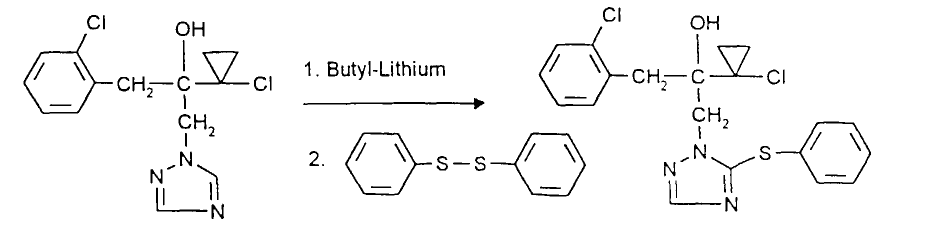

Verwendet man 2-(1-Chlor-cyclopropyl)-1-(2-chlorphenyl)-3-(5-phenylthio-1,2,4-triazol-l-yl)-propan-2-ol und setzt dieses mit einer äquimolaren Menge an Wasserstoffperoxid als Oxidationsmittel um, so kann der Verlauf der zweiten Stufe des erfindungsgemäßen Verfahrens (b) durch das folgende Formelschema veranschaulicht werden:

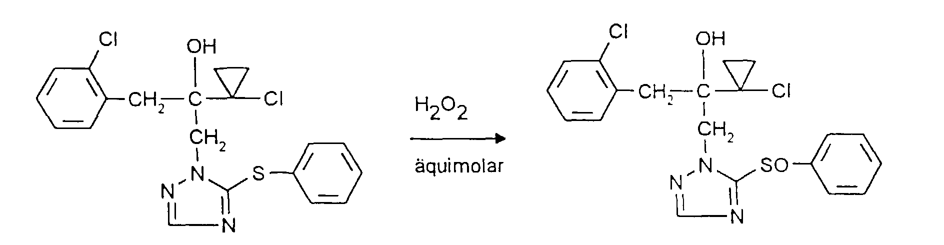

Verwendet man 2-(1-Chlor-cyclopropyl)-1-(2-chlorphenyl)-3-(5-mercapto-1,2,4-triazol-1-yl)-propan-2-ol als Ausgangsstoff und Kaliumpermanganat als Oxidationsmittel, so kann der Verlauf des erfindungsgemäßen Verfahrens (c) durch das folgende Formelschema veranschaulicht werden:

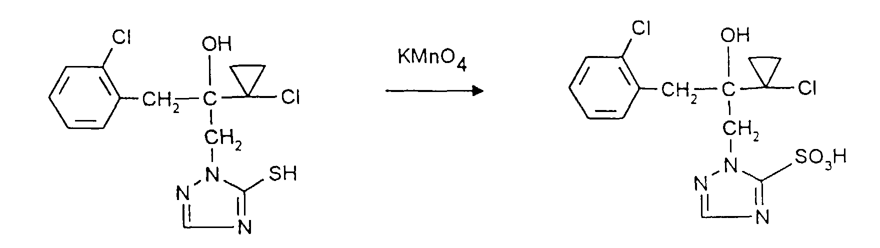

Die bei der Durchführung des erfindungsgemäßen Verfahrens (a) als Ausgangsstoffe benötigten Hydroxyethyl-triazole sind durch die Formel (II) allgemein definiert. In dieser Formel haben R¹ und R² vorzugsweise diejenigen Bedeutungen, die bereits im Zusammenhang mit der Beschreibung der erfindungsgemäßen Stoffe der Formel (I) vorzugsweise für diese Reste genannt wurden.

Die Hydroxyethyl-triazole der Formel (II) sind bekannt oder lassen sich nach bekannten Methoden herstellen (vgl. EP-A 0 015 756, EP-A 0 040 345, EP-A 0 052 424, EP-A 0 061 835, EP-A 0 297 345 und EP-A 0 470 463).

Als Basen kommen bei der Durchführung der ersten Stufe des erfindungsgemäßen Verfahrens (a), Variante (α), alle für derartige Reaktionen üblichen, starken Alkalimetall-Basen in Betracht. Vorzugsweise verwendbar sind n-Butyl-lithium, Lithium-diisopropyl-amid, Natriumhydrid, Natriumamid und auch Kalium-tert.-butylat im Gemisch mit Tetramethylethylen-diamin (= TMEDA).

Bei der Durchführung der ersten Stufe des erfindungsgemäßen Verfahrens (a), Variante (α), kommen alle für derartige Umsetzungen üblichen inerten organischen Solventien als Verdünnungsmittel in Betracht. Vorzugsweise verwendbar sind Ether, wie Tetrahydrofuran, Dioxan, Diethylether und 1,2-Dimethoxyethan, ferner flüssiger Ammoniak oder auch stark polare Solventien, wie Dimethylsulfoxid.

Schwefel wird vorzugsweise in Form von Pulver eingesetzt. Zur Hydrolyse verwendet man bei der Durchführung der ersten Stufe des erfindungsgemäßen Verfahrens (a), Variante (α), Wasser, gegebenenfalls in Gegenwart einer Säure. In Frage kommen hierbei alle für derartige Umsetzungen üblichen anorganischen oder organischen Säuren. Vorzugsweise verwendbar sind Essigsäure, verdünnte Schwefelsäure und verdünnte Salzsäure. Es ist jedoch auch möglich, die Hydrolyse mit wäßriger Ammoniumchlorid-Lösung durchzuführen.

Die Reaktionstemperaturen können bei der Durchführung der ersten Stufe des erfindungsgemäßen Verfahrens (a), Variante (α), innerhalb eines bestimmten Bereiches variiert werden. Im allgemeinen arbeitet man bei Temperaturen zwischen -70°C und +20°C, vorzugsweise zwischen -70°C und 0°C.

Bei der Durchführung aller Schritte des erfindungsgemäßen Verfahrens (a) arbeitet man im allgemeinen unter Normaldruck. Es ist aber auch möglich, unter erhöhtem oder vermindertem Druck zu arbeiten. So kommt vor allem bei der Durchführung der ersten Stufe des erfindungsgemäßen Verfahrens (a) nach Variante (α) ein Arbeiten unter erhöhtem Druck in Frage.

Bei der Durchführung der ersten Stufe des erfindungsgemäßen Verfahrens (a) nach Variante (α) setzt man auf 1 Mol an Hydroxyethyl-triazol der Formel (II) im allgemeinen 2 bis 3 Äquivalente, vorzugsweise 2,0 bis 2,5 Äquivalente, an starker Base und anschließend eine äquivalente Menge oder auch einen Überschuß an Schwefel ein. Die Umsetzung kann unter Schutzgas-atmosphäre, z.B. unter Stickstoff oder Argon, vorgenommen werden. Die Aufarbeitung erfolgt nach üblichen Methoden. Im allgemeinen geht man so vor, daß man das Reaktionsgemisch mit einem in Wasser wenig löslichen organischen Solvens extrahiert, die vereinigten organischen Phasen trocknet und einengt und den verbleibenden Rückstand gegebenenfalls durch Umkristallisation und/oder Chromatographie reinigt.

Bei der Durchführung der ersten Stufe des erfindungsgemäßen Verfahrens (a) nach Variante (β) kommen als Verdünnungsmittel alle für derartige Umsetzungen üblichen, hoch siedenden organischen Solventien in Betracht. Vorzugsweise verwendbar sind Amide, wie Dimethylformamid und Dimethylacetamid, außerdem heterocyclische Verbindungen, wie N-Methyl-pyrrolidon, und auch Ether, wie Diphenylether.

Schwefel wird auch bei der Durchführung der ersten Stufe des erfindungsgemäßen Verfahrens (a) nach der Variante (β) im allgemeinen in Form von Pulver eingesetzt. Nach der Umsetzung kann gegebenenfalls eine Behandlung mit Wasser sowie gegebenenfalls mit Säure vorgenommen werden. Diese wird so durchgeführt wie die Hydrolyse bei der Durchführung der ersten Stufe des erfindungsgemäßen Verfahrens (a) nach der Variante (α).

Die Reaktionstemperaturen können auch bei der Durchführung der ersten Stufe des erfindungsgemäßen Verfahrens (a) nach Variante (β) innerhalb eines größeren Bereiches variiert werden. Im allgemeinen arbeitet man bei Temperaturen zwischen 150°C und 300°C, vorzugsweise zwischen 180°C und 250°C.

Bei der Durchführung der ersten Stufe des erfindungsgemäßen Verfahrens (a) nach Variante (β) setzt man auf 1 Mol an Hydroxyethyl-triazol der Formel (II) im allgemeinen 1 bis 5 Mol, vorzugsweise 1,5 bis 3 Mol an Schwefel ein. Die Aufarbeitung erfolgt nach üblichen Methoden. Im allgemeinen geht man so vor, daß man das Reaktionsgemisch mit einem in Wasser nur wenig löslichen organischen Solvens extrahiert, die vereinigten organischen Phasen trocknet und einengt und den verbleibenden Rückstand gegebenenfalls nach üblichen Methoden, wie Umkristallisation oder Chromatographie, von eventuell vorhandenen Verunreinigungen befreit.

Die bei der Durchführung der zweiten Stufe des erfindungsgemäßen Verfahrens (a) als Ausgangssubstanzen benötigten Verbindungen der Formel (Ia) sind erfindungsgemäße Stoffe.

Die bei der Durchführung des erfindungsgemäßen Verfahrens (a) in der zweiten Stufe als Reaktionskomponenten benötigten Halogen-Verbindungen sind durch die Formel (III) allgemein definiert.
- R⁴: steht bevorzugt für geradkettiges oder verzweigtes Alkyl mit 1 bis 4 Kohlenstoffatomen, wobei jeder dieser Reste einfach bis dreifach durch Fluor und/oder Chlor substituiert sein kann,
oder
für geradkettiges oder verzweigtes Alkenyl mit 2 bis 5 Kohlenstoffatomen, wobei jeder dieser Reste einfach bis dreifach, gleichartig oder verschieden durch Fluor und/oder Chlor substituiert sein kann,
oder
für Phenylalkyl mit 1 bis 3 Kohlenstoffatomen im geradkettigen oder verzweigten Alkylteil, wobei jeder dieser Reste im Phenylteil einfach bis dreifach, gleichartig oder verschieden substituiert sein kann durch Fluor, Chlor, Brom, Methyl, Ethyl, tert.-Butyl, Trichlormethyl und/oder Trifluormethyl.
- R⁴: steht besonders bevorzugt für Methyl, Ethyl oder Propyl, wobei jeder dieser Reste einfach bis dreifach, gleichartig oder verschieden durch Fluor und/oder Chlor substituiert sein kann,
oder
für Allyl, But-2-en-yl oder But-3-enyl, wobei jeder dieser Reste einfach bis dreifach, gleichartig oder verschieden substituiert sein kann durch Fluor und/oder Chlor,
oder
für Phenylalkyl mit 1 bis 2 Kohlenstoffatomen im geradkettigen oder verzweigten Alkylteil, wobei jeder dieser Reste im Phenylteil einfach oder zweifach substituiert sein kann durch Fluor, Chlor, Brom, Methyl, Ethyl, tert.-Butyl, Trichlormethyl und/oder Trifluormethyl.

Hal steht auch vorzugsweise für Chlor, Brom oder Iod.

Die Halogen-Verbindungen der Formel (III) sind bekannt.

Als Säurebindemittel kommen bei der Durchführung der zweiten Stufe des erfindungsgemäßen Verfahrens (a) alle üblichen anorganischen oder organischen Basen in Frage. Vorzugsweise verwendbar sind Erdalkali- oder Alkalimetallhydroxide wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Als Verdünnungsmittel kommen bei der Durchführung der zweiten Stufe des erfindungsgemäßen Verfahrens (a) alle für derartige Umsetzungen üblichen, inerten organischen Solventien in Betracht. Vorzugsweise verwendbar sind Ether, wie Diethylether, Methyl-tert.-butyl-ether, Ethylenglykol-dimethylether, Tetrahydrofuran und Dioxan, ferner Nitrile, wie Acetonitril, und außerdem stark polare Solventien, wie Dimethylsulfoxid oder Dimethylformamid.

Die Reaktionstemperaturen können bei der Durchführung der zweiten Stufe des erfindungsgemäßen Verfahrens (a) innerhalb eines größeren Bereiches variiert werden. Im allgemeinen arbeitet man bei Temperaturen zwischen 0°C und 120°C, vorzugsweise zwischen 20°C und 100°C.

Bei der Durchführung der zweiten Stufe des erfindungsgemäßen Verfahrens (a) setzt man auf 1 Mol an Triazolyl-Derivat der Formel (Ia) im allgemeinen 1 bis 2 Mol an Halogen-Verbindung der Formel (III) sowie eine äquivalente Menge oder auch einen Überschuß an Säurebindemittel ein. Die Aufarbeitung erfolgt nach üblichen Methoden. Im allgemeinen geht man so vor, daß man das Reaktionsgemisch mit wäßriger Base und einem mit Wasser wenig mischbaren organischen Lösungsmittel versetzt, die organische Phase abtrennt, trocknet und einengt. Das erhaltene Produkt kann gegebenenfalls nach üblichen Methoden, z.B. durch Umkristallisation, von noch vorhandenen Verunreinigungen befreit werden.

Die bei der Durchführung der dritten Stufe des erfindungsgemäßen Verfahrens (a) als Ausgangssubstanzen benötigten Verbindungen der Formel (Ib) sind erfindungsgemäße Stoffe.

Als Oxidationsmittel kommen bei der Durchführung der dritten Stufe des erfindungsgemäßen Verfahrens (a) alle zur Oxidation von Schwefel üblichen Substanzen in Frage. Vorzugsweise verwendbar sind Wasserstoffperoxid und Persäuren, wie Peressigsäure und meta-Chlor-perbenzoesäure, und außerdem anorganische Salze, wie Kaliumpermanganat.

Als Verdünnungsmittel kommen bei der Durchführung der dritten Stufe des erfindungsgemäßen Verfahrens (a) alle für derartige Umsetzungen üblichen Solventien in Betracht. Verwendet man Wasserstoffperoxid oder Persäuren als Oxidationsmittel, so setzt man vorzugsweise Essigsäure oder Eisessig als Verdünnungsmittel ein. Arbeitet man mit Kaliumpermanganat als Oxidationsmittel, so kommen vorzugsweise Wasser oder Alkohole, wie tert.-Butanol, als Solventien in Frage.

Die Reaktionstemperaturen können bei der Durchführung der dritten Stufe des erfindungsgemäßen Verfahrens (a) innerhalb eines bestimmten Bereiches variiert werden. Im allgemeinen arbeitet man bei Temperaturen zwischen 0°C und 100°C, vorzugsweise zwischen 10°C und 100°C.

Bei der Durchführung der dritten Stufe des erfindungsgemäßen Verfahrens (a) setzt man auf 1 Mol an Verbindung der Formel (Ib) im allgemeinen eine äquivalente Menge oder einen Überschuß an Oxidationsmittel ein. Ist die Herstellung von SO-Verbindungen gewünscht, so arbeitet man im allgemeinen mit äquimolaren Mengen. Ist die Synthese von SO₂-Verbindungen beabsichtigt, so wählt man einen Überschuß an Oxidationsmitteln. Die Aufarbeitung erfolgt nach üblichen Methoden. Im allgemeinen geht man so vor, daß man mit Eis oder Wasser verdünnt, gegebenenfalls durch Zugabe von Base alkalisch stellt, mit einem mit Wasser wenig mischbaren, organischen Solvens extrahiert, die vereinigten organischen Phasen trocknet und einengt und das entstehende Produkt gegebenenfalls umkristallisiert. Arbeitet man mit Kaliumpermanganat in wäßriger Lösung, so verfährt man im allgemeinen in der Weise, daß man den Feststoff abfiltriert, wäscht und trocknet.

Die bei der Durchführung der ersten Stufe des erfindungsgemäßen Verfahrens (b) als Reaktionskomponenten benötigten Diaryldisulfide sind durch die Formel (IV) allgemein definiert.
- R⁵: steht bevorzugt für Phenyl, das einfach bis dreifach, gleichartig oder verschieden substituiert sein kann durch Fluor, Chlor, Brom, Methyl, Ethyl, tert.-Butyl, Trichlormethyl und/oder Trifluormethyl.
- R⁵: steht besonders bevorzugt für Phenyl, das einfach oder zweifach substituiert sein kann durch Fluor, Chlor, Brom, Methyl, Ethyl, tert.-Butyl, Trichlormethyl und/oder Trifluormethyl.

Die Diaryl-disulfide der Formel (IV) sind bekannt oder lassen sich nach bekannten Methoden herstellen.

Als starke Basen kommen bei der Durchführung der ersten Stufe des erfindungsgemäßen Verfahrens (b) alle diejenigen starken Basen in Betracht, die bereits im Zusammenhang mit der Beschreibung der ersten Stufe des erfindungsgemäßen Verfahrens (a) genannt wurden.

Als Verdünnungsmittel kommen bei der Durchführung der ersten Stufe des erfindungsgemäßen Verfahrens (b) alle diejenigen Solventien in Betracht, die schon im Zusammenhang mit der Beschreibung der ersten Stufe des erfindungsgemäßen Verfahrens (a) genannt wurden.

Auch die übrigen Reaktionsbedingungen und die Aufarbeitungsmethoden bei der Durchführung der ersten Stufe des erfindungsgemäßen Verfahrens (b) entsprechen denjenigen, die bereits im Zusammenhang mit der Beschreibung der ersten Stufe des erfindungsgemäßen Verfahrens (a) genannt wurden.

Bei der Durchführung der zweiten Stufe des erfindungsgemäßen Verfahrens (b) kommen als Oxidationsmittel alle diejenigen Oxidantien in Betracht, die schon im Zusammenhang mit der Beschreibung der dritten Stufe des erfindungsgemäßen Verfahrens (a) genannt wurden.

Auch die Reaktionsbedingungen und die Aufarbeitungsmethoden sind bei der Durchführung der zweiten Stufe des erfindungsgemäßen Verfahrens (b) analog zu denjenigen, die schon im Zusammenhang mit der Beschreibung der dritten Stufe des erfindungsgemäßen Verfahrens (a) genannt wurden. Gleiches gilt für die Durchführung des erfindungsgemäßen Verfahrens (c).

Die nach den erfindungsgemäßen Verfahren erhältlichen Triazolyl-Derivate der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung unerwünschter Mikroorganismen, wie Fungi und Bakterien, im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Pyricularia oryzae und Pellicularia sasakii an Reis sowie zur Bekämpfung von Getreidekrankheiten, wie Pseudocercosporella, Erysiphe- und Fusarium-Arten. Außerdem lassen sich die erfindungsgemäßen Stoffe sehr gut gegen Venturia und Sphaerotheca einsetzen. Sie besitzen darüber hinaus auch eine sehr gute in-vitro Wirkung.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannte :
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Verwendung im Pflanzenschutz als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen beispielsweise die folgenden Stoffe infrage.

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid;(E)-2-Methoxyimino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat;Methyl-(E)-methoximino [alpha-(o-tolyloxy)-o-tolyl]-acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Die Herstellung und die Verwendung der erfindungsgemäßen Stoffe gehen aus den folgenden Beispielen hervor.

### Herstellungsbeispiele

### Beispiel 1

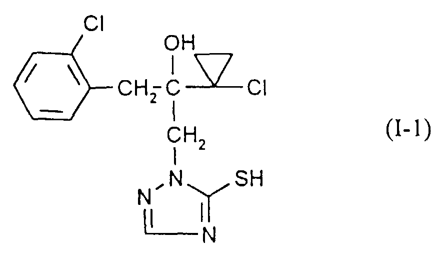

### Variante α:

Ein Gemisch aus 3,12 g (10 mMol) 2-(1-Chlor-cyclopropyl)-1-(2-chlorphenyl)-3-(1,2,4-triazol-1-yl)-propan-2-ol und 45 ml absolutem Tetrahydrofuran wird bei-20°C mit 8,4 ml (21 mMol) n-Butyl-lithium in Hexan versetzt und 30 Minuten bei 0°C gerührt. Danach wird das Reaktionsgemisch auf -70°C abgekühlt, mit 0,32 g (10 mMol) Schwefel-Pulver versetzt und 30 Minuten bei -70°C gerührt. Es wird auf -10°C erwärmt, mit Eiswasser versetzt und durch Zugabe von verdünnter Schwefelsäure auf einen pH-Wert von 5 eingestellt. Man extrahiert mehrfach mit Essigsäureethylester, trocknet die vereinigten organischen Phasen über Natriumsulfat und engt unter vermindertem Druck ein. Man erhält auf diese Weise 3,2 g (93 % der Theorie) an 2-(1-Chlor-cyclopropyl)-1-(2-chlorphenyl)-3-(5-mercapto-1,2,4-triazol-1-yl)-propan-2-ol in Form einer Festsubstanz, die nach Umkristallisation bei 138-139°C schmilzt.

### Variante β:

Ein Gemisch aus 3,12 g (10 mmol) 2-(1-Chlor-cyclopropyl)-1-(2-chlor-phenyl)-3-(1,2,4-triazol-1-yl)-propan-2-ol, 0,96 g (30 mmol) Schwefel-Pulver und 20 ml absolutem N-Methyl-pyrrolidon wird unter Rühren 44 Stunden auf 200°C erhitzt. Anschließend wird das Reaktionsgemisch unter vermindertem Druck (0,2 mbar) eingeengt. Das dabei anfallende Rohprodukt (3,1 g) wird aus Toluol umkristallisiert. Man erhält auf diese Weise 0,7 g (20 % der Theorie) an 2-(1-Chlor-cyclopropyl)-1(2-chlorphenyl)-3-(5-mercapto-1,2,4-triazol-1-yl)-propan-2-ol in Form einer Festsubstanz vom Schmelzpunkt 138-139°C.

### Beispiel 2

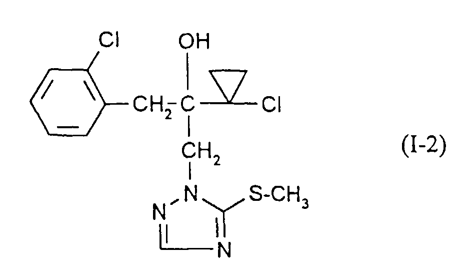

Ein Gemisch aus 3,43 g (10 mMol) 2-(1-Chlor-cyclopropyl)-1-(2-chlorphenyl)-3-(5-mercapto-1,2,4-triazol-1-yl)-propan-2-ol, 20 ml absolutem Acetonitril und 1,38 g (10 mMol) Kaliumcarbonat wird mit 0,93 ml (15 mMol) Methyliodid versetzt und 5 Stunden bei 40°C gerührt. Danach wird das Reaktionsgemisch mit gesättigter, wäßriger Natriumcarbonat-Lösung versetzt und mehrfach mit Essigsäureethylester extrahiert. Die vereinigten organischen Phasen werden über Natriumsulfat getrocknet und unter vermindertem Druck eingeengt. Man erhält auf diese Weise 3,4 g (95 % der Theorie) an 2-(1-Chlor-cyclopropyl)-1-(2-chlorphenyl)-3-(5-methylthio-1,2,4-triazol-1-yl)-propan-2-ol in Form eines Öles.
¹H-NMR-Spektrum (200 MHz; CDCl₃, TMS):
- δ =: 0,6-1,05 (m, 4H); 2,7 (s, 3H); 3,35 (AB, 2H); 4,4 (AB, 2H); 4,7 (OH); 7,2-7,6 (m, 4H); 7,9 (s, 1H).

### Beispiel 3

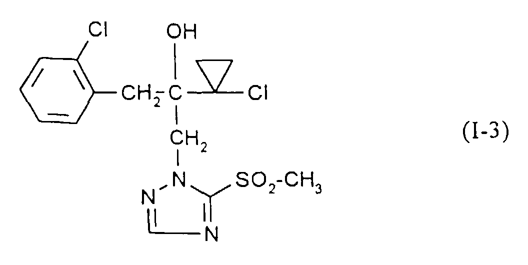

Eine Lösung von 3,57 g (10 mMol) 2-(1-Chlorcyclopropyl)-1-(2-chlorphenyl)-3-(5-methylthio-1,2,4-triazol-1-yl)-propan-2-ol in 40 ml Eisessig wird bei 90°C unter Rühren tropfenweise mit 4 ml wäßriger Wasserstoffperoxid-Lösung (35 %ig) versetzt. Das Reaktionsgemisch wird nach beendeter Zugabe noch 30 Minuten bei 90°C gerührt, dann auf Raumtemperatur abgekühlt, mit Eis versetzt und durch Hinzufügen von wäßriger Natronlauge alkalisch eingestellt. Man extrahiert mehrfach mit Essigsäureethylester, trocknet die vereinigten organischen Phasen über Natriumsulfat und engt unter vermindertem Druck ein. Das dabei verbleibende, langsam kristallisierende Produkt wird abgesaugt. Man erhält auf diese Weise 2,0 g (51 % der Theorie) an 2-(1-Chlor-cyclopropyl)-1-(2-chlorphenyl)-3-(5-methylsulfonyl-1,2,4-triazol-1-yl)-propan-2-ol in Form einer Festsubstanz, die bei 125-128°C schmilzt.

### Beispiel 4

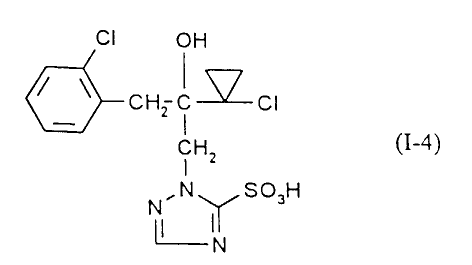

Ein Gemisch aus 1,71 g (5 mMol) 2-(1-Chlorcyclopropyl)-1-(2-chlorphenyl)-3-(5-mercapto-1,2,4-triazol-1-yl)-propan-2-ol, 1,58 g (10 mMol) Kaliumpermanganat und 20 ml Wasser wird 30 Minuten bei Raumtemperatur gerührt. Danach wird der Feststoff abgesaugt, mit Wasser gewaschen und getrocknet. Man erhält auf diese Weise 2,0 g (100 % der Theorie) an 2-(1-Chlor-cyclopropyl)-1-(2-chlorphenyl)-3-(5-sulfo-1,2,4-triazol-1-yl)-propan-2-ol in Form einer Festsubstanz, die bei 68-70°C schmilzt.

### Beispiel 5

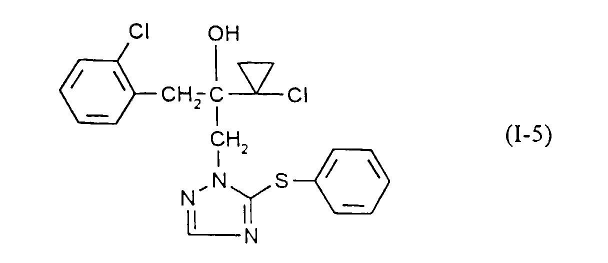

Ein Gemisch aus 3,12 g (10 mMol) 2-(1-Chlor-cyclopropyl)-1-(2-chlorphenyl)-3-(1,2,4-triazol-1-yl)-propan-2-ol und 45 ml absolutem Tetrahydrofuran wird bei - -20°C mit 8,4 ml (21 mMol) n-Butyl-lithium in Hexan versetzt und 30 Minuten bei 0°C gerührt. Danach wird das Reaktionsgemisch auf -70°C abgekühlt, mit 2,18 g (10 mMol) Diphenyl-disulfid versetzt und unter Rühren langsam auf Raumtemperatur aufgetaut. Man rührt noch weitere 19 Stunden bei Raumtemperatur, verdünnt mit Essigsäureethylester und schüttelt mehrfach mit gesättigter, wäßriger Natriumcarbonat-Lösung aus. Die organische Phase wird über Natriumsulfat getrocknet und unter vermindertem Druck eingeengt. Der verbleibende Rückstand von 4,2 g wird mit einem Gemisch Petrolether/Essigsäureethylester = 2:1 über 500 g Kieselgel chromatographiert. Nach dem Eindampfen des Eluates erhält man 3,5 g (84 % der Theorie) an 2-(1-Chlor-cyclopropyl)-1-(2-chlorphenyl)-3-(5-phenylthio-1,2,4-triazol-1-yl)-propan-2-ol in Form eines Öles.
Massenspektrum (Cl): 420 (M+H⁺)

Nach den zuvor angegebenen Methoden werden auch die in der folgenden Tabelle 2 aufgeführten Stoffe hergestellt.

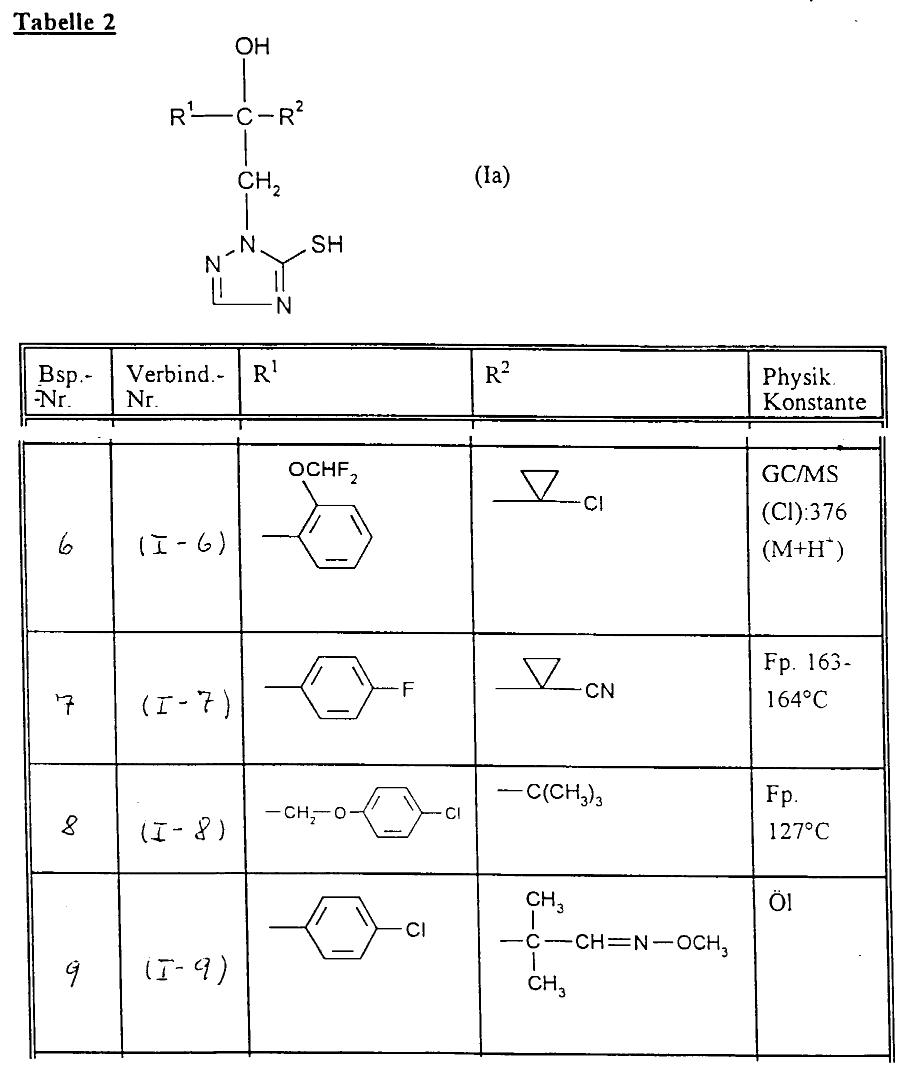

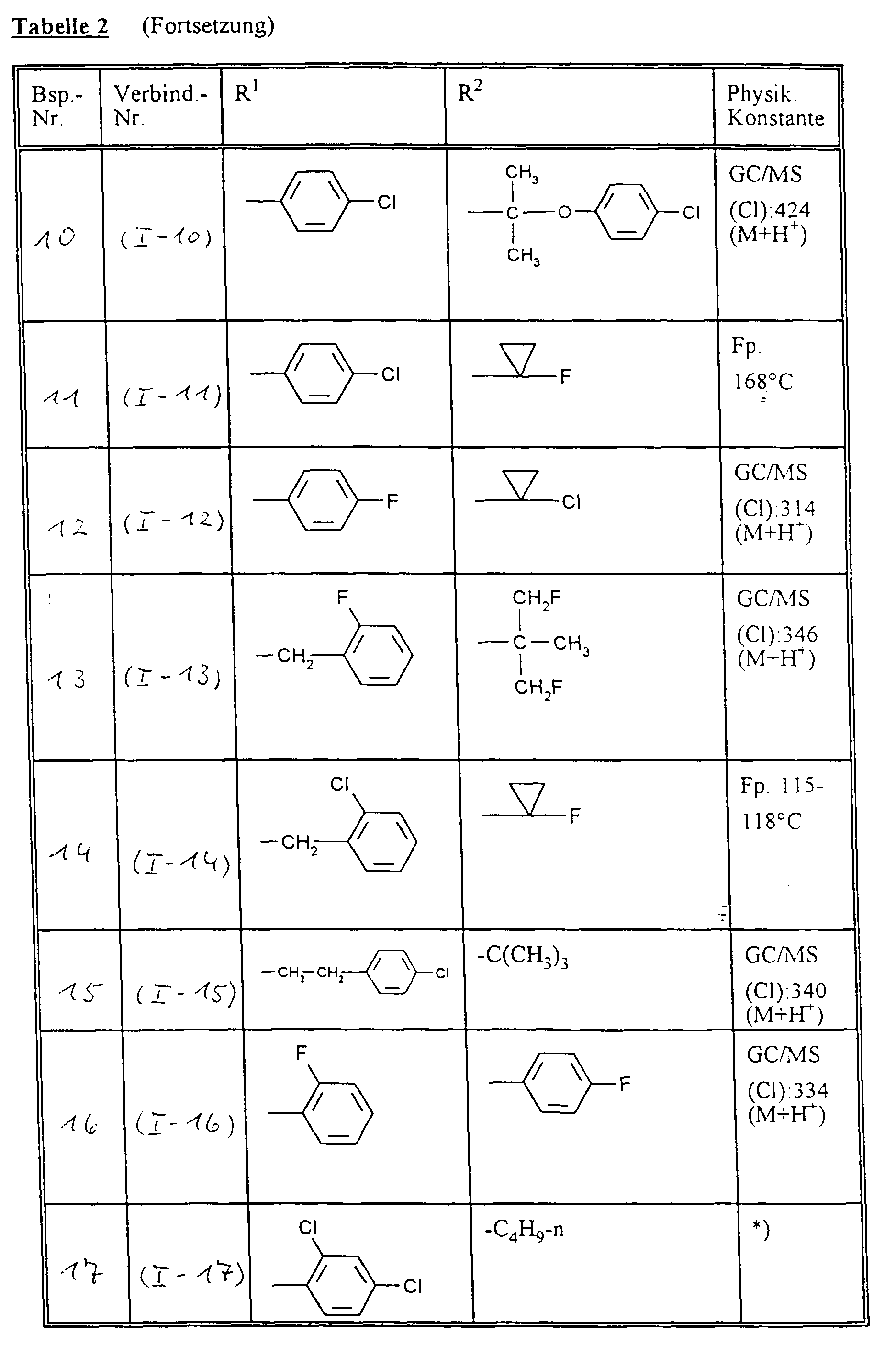

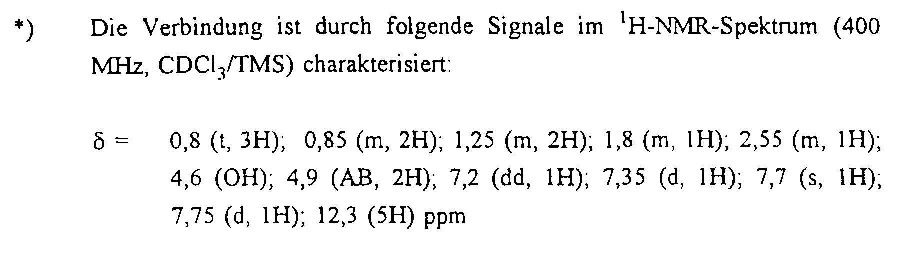

### Verwendungsbeispiele

### Beispiel A

### Erysiphe-Test (Gerste) / protektiv

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Wirkstoffe, Wirkstoffkonzentrationen und Versuchsergebnisse gehen aus der folgenden Tabelle hervor.

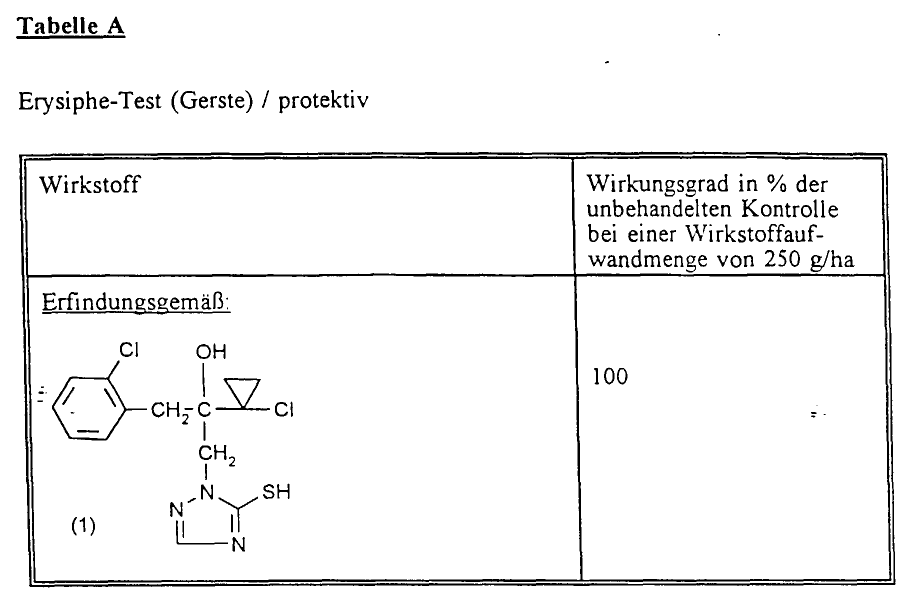

### Beispiel B

### Erysiphe-Test (Weizen) / protektiv

- Lösungsmittel:: 10 Gewichtsteile N-Methyl-pyrrolidon
- Emulgator:: 10 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Menge. Nach Antrocknen des Spritzbelages werden die Pflanzen mit Sporen von Erysiphe graminis f.sp.tritici bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Wirkstoffe, Wirkstoffkonzentrationen und Versuchsergebnisse gehen aus der folgenden Tabelle hervor.

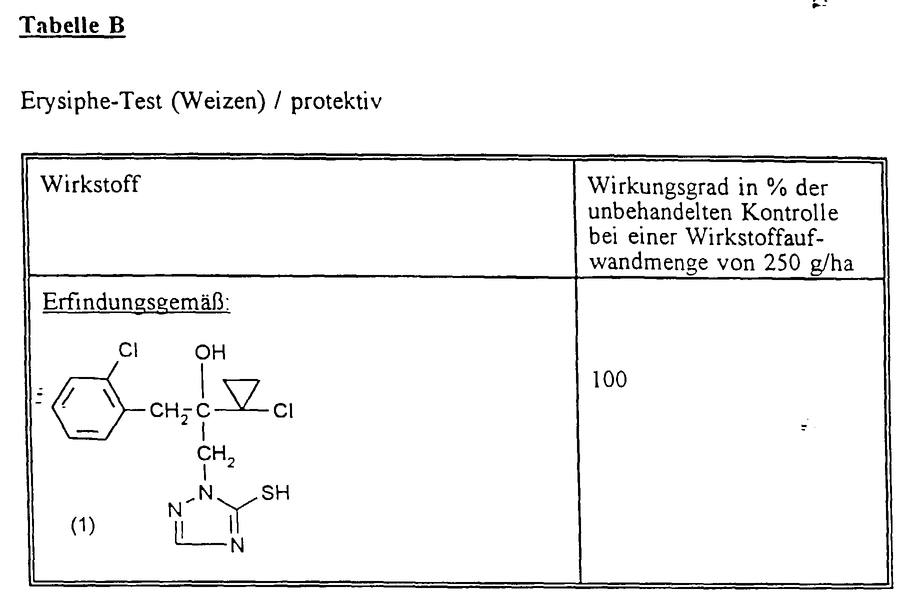

### Beispiel C

### Pseudocercosporella herpotrichoides-Test (Weizen) / protektiv

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen an der Halmbasis mit Sporen von Pseudocercosporella herpotrichoides inokuliert.

Die Pflanzen werden in einem Gewächshaus bei einer Temperatur von ca. 10°C und einer relativen Luftfeuchtigkeit von ca. 80 % aufgestellt.

21 Tage nach der Inokulation erfolgt die Auswertung.

Wirkstoffe, Wirkstoffkonzentrationen und Versuchsergebnisse gehen aus der folgenden Tabelle hervor.

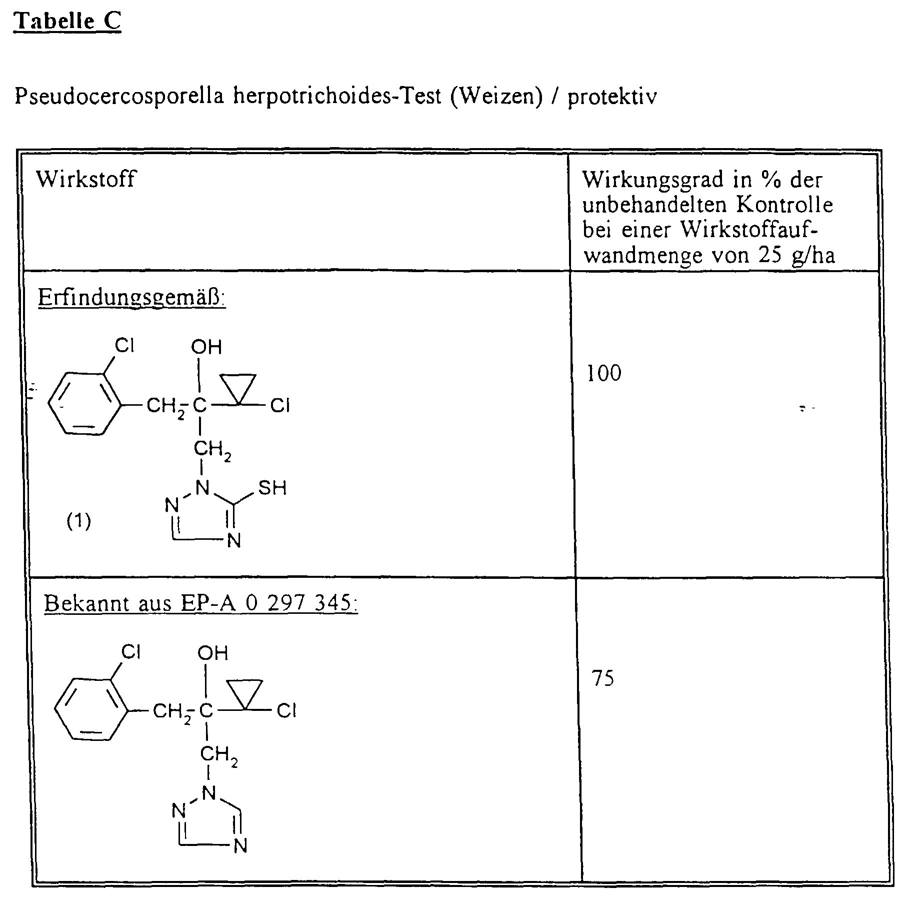

### Beispiel D

### Fusarium nivale (var. nivale) - Test (Weizen) / protektiv

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit einer Konidiensuspension von Fusarium nivale var. nivale besprüht.

Die Pflanzen werden in einem Gewächshaus unter lichtdurchlässigen Inkubationshauben bei einer Temperatur von ca. 15°C und einer relativen Luftfeuchtigkeit von ca. 100 % aufgestellt.

Wirkstoffe, Wirkstoffkonzentrationen und Versuchsergebnisse gehen aus der folgenden Tabelle hervor.

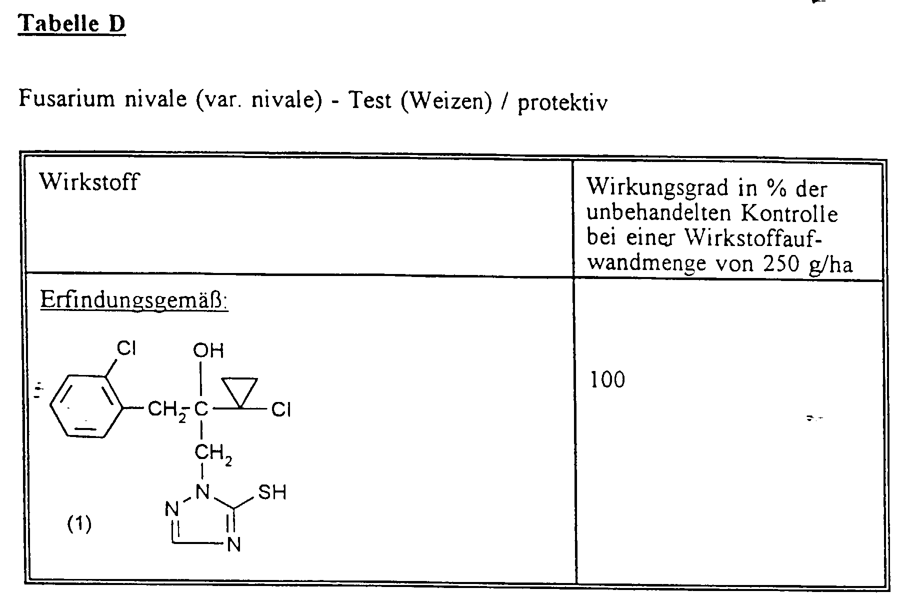

### Beispiel E

### Fusarium culmorum-Test (Weizen) / protektiv

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit einer Konidiensuspension von Fusarium culmorum besprüht.

Die Pflanzen werden in einem Gewächshaus unter lichtdurchlässigen Inkubationshauben bei einer Temperatur von ca. 20°C und einer relativen Luftfeuchtigkeit von ca. 100 % aufgestellt.

4 Tage nach der Inokulation erfolgt die Auswertung.

Wirkstoffe, Wirkstoffkonzentrationen und Versuchsergebnisse gehen aus den folgenden Tabellen hervor.

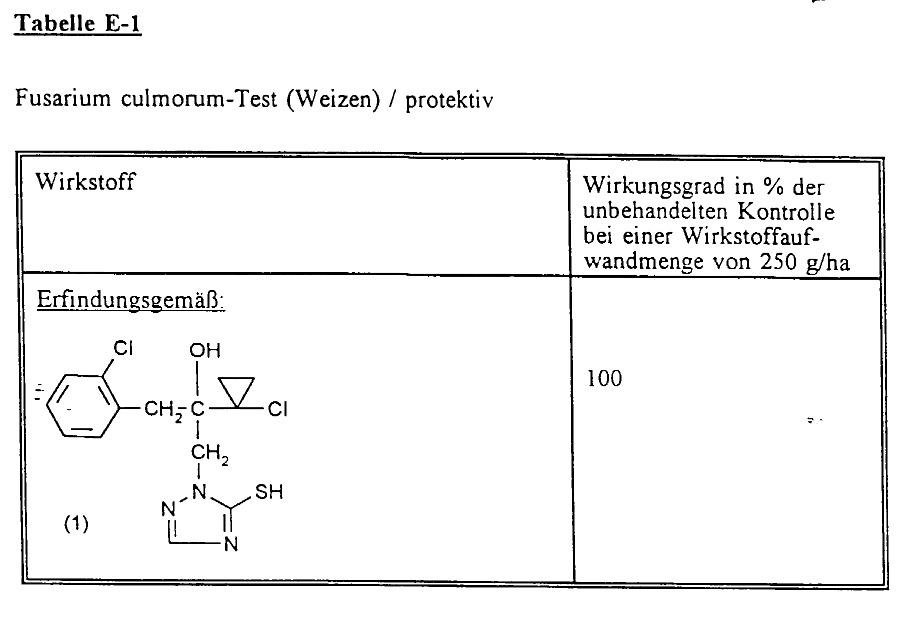

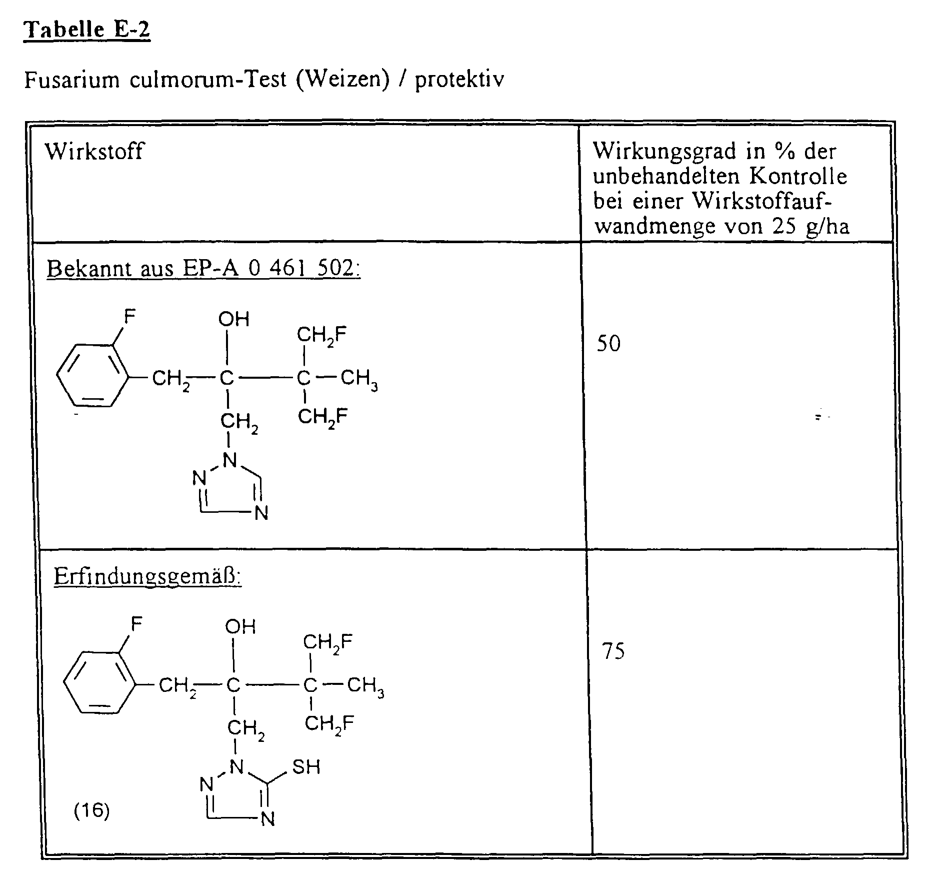

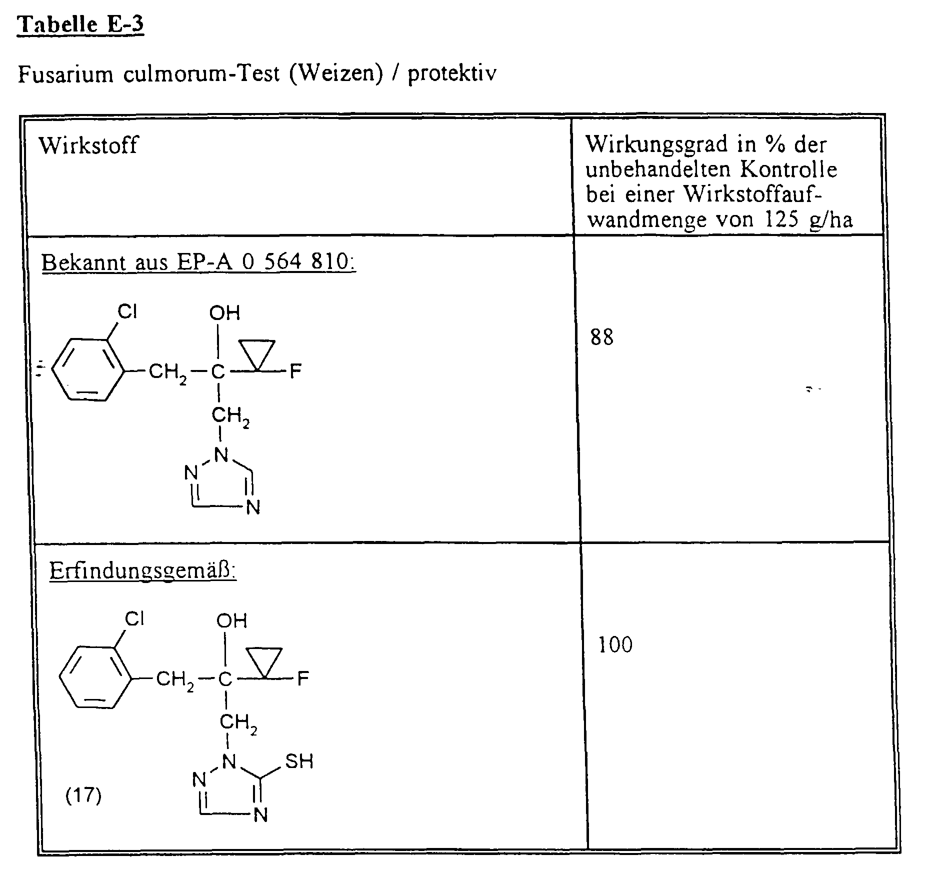

### Beispiel F

### Pellicularia-Test (Reis)

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Antrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles

Wirkstoffe, Wirkstoffkonzentrationen und Versuchsergebnisse gehen aus der folgenden Tabelle hervor.

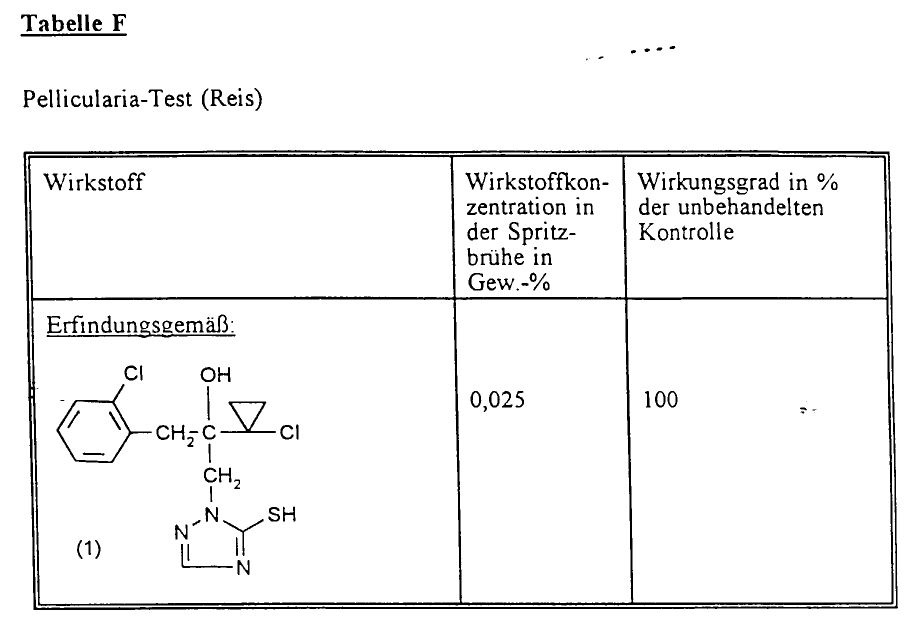

### Beispiel G

### Sphaerotheca-Test (Gurke) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelagess werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

Wirkstoffe, Wirkstoffkonzentrationen und Versuchsergebnisse gehen aus der folgenden Tabelle hervor.

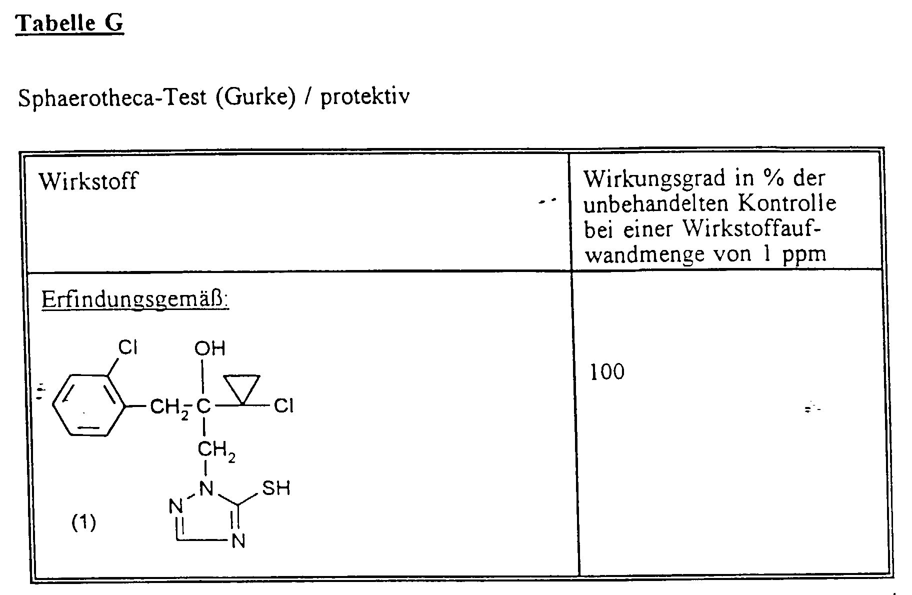

## Patentansprüche

1. Triazolyl-Derivate der Formel 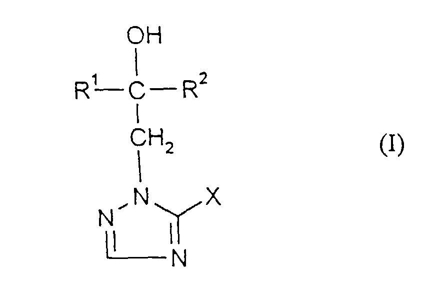 in welcher
R¹ für geradkettiges oder verzweigtes Alkyl mit 1 bis 6 Kohlenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
R¹ für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
R¹ für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
R¹ für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
R¹ für Aryl mit 6 bis 10 Kohlenstoffatomen steht, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
R¹ für einen gegebenenfalls benzanellierten fünf- oder sechsgliedrigen heteroaromatischen Rest mit 1 bis 3 Heteroatomen steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano,
R² für geradkettiges oder verzweigtes Alkyl mit 1 bis 6 Kohlenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
R² für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
R² für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
R² für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
R² für Aryl mit 6 bis 10 Kohlenstoffatomen steht, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
R² für einen gegebenenfalls benzanellierten fünf- oder sechsgliedrigen heteroaromatischen Rest mit 1 bis 3 Heteroatomen steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxy-. alkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano,
und
X für die Gruppierungen -SH, -SR³, -SO-R³, -SO₂-R³ oder -SO₃H steht, worin
R³ für geradkettiges oder verzweigtes Alkyl mit 1 bis 6 Kohlenstoffatomen steht, wobei jeder dieser Reste einfach bis dreifach durch Fluor und/oder Chlor substituiert sein kann,
oder
R³ für geradkettiges oder verzweigtes Alkenyl mit 2 bis 6 Kohlenstoffatomen steht, wobei jeder dieser Reste einfach bis dreifach durch Fluor und/oder Chlor substituiert sein kann,
oder
R³ für Phenylalkyl mit 1 bis 4 Kohlenstoffatomen im geradkettigen oder verzweigten Alkylteil steht, wobei jeder dieser Reste im Phenylteil einfach bis dreifach, gleichartig oder verschieden substituiert sein kann durch Halogen, Alkyl mit 1 bis 4 Kohlenstoffatomen und/oder Halogenalkyl mit 1 bis 4 Kohlenstoffatomen und 1 bis 5 Halogenatomen,
oder
R³ für Phenyl steht, das einfach bis dreifach, gleichartig oder verschieden substituiert sein kann durch Halogen, Alkyl mit 1 bis 4 Kohlenstoffatomen und/oder Halogenalkyl mit 1 bis 4 Kohlenstoffatomen und 1 bis 5 Halogenatomen,
sowie deren Säureadditions-Salze und Metallsalz-Komplexe.

2. Triazolyl-Derivate der Formel (I) gemäß Anspruch 1, in welcher
R¹ für 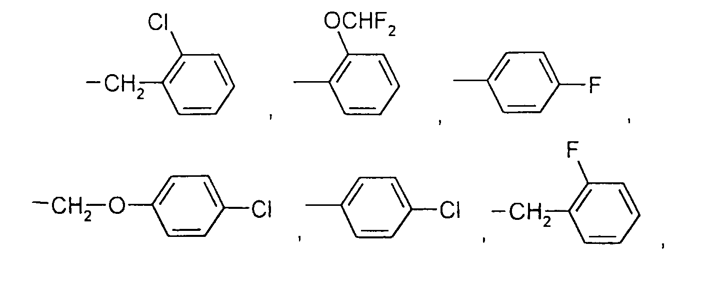 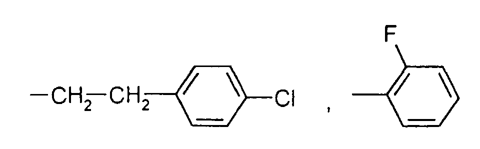 oder 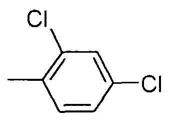 steht,
R² für 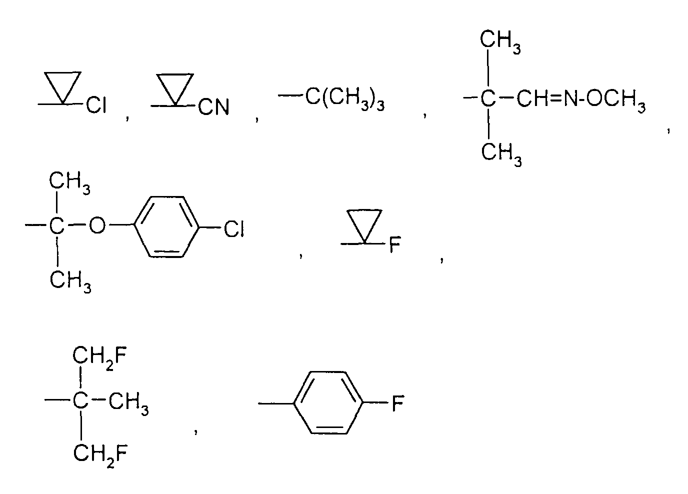 oder -C₄H₉-n steht und
X für -SH, -SCH₃, -SO₂CH₃, -SO₃H oder 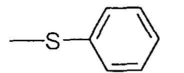 steht.

3. Triazolyl-Derivat gemäß Anspruch 1, gekennzeichnet durch die Formel

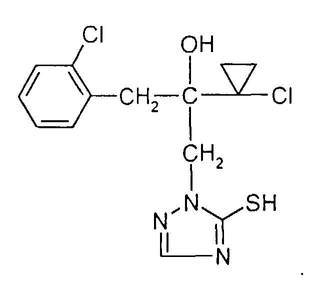

4. Verfahren zur Herstellung von Triazolyl-Derivaten der Formel (I) gemäß Anspruch 1
sowie von deren Säureadditions-Salzen und Metallsalz-Komplexen, dadurch gekennzeichnet, daß man
a) Hydroxyethyl-triazole der Formel 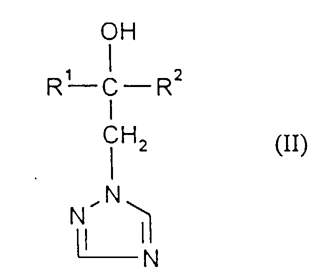 in welcher
R¹ und R² die oben angegebenen Bedeutungen haben,
entweder
α) nacheinander mit starken Basen und Schwefel in Gegenwart eines Verdünnungsmittels umsetzt und dann mit Wasser, gegebenenfalls in Gegenwart einer Säure hydrolysiert,
oder
β) mit Schwefel in Gegenwart eines hoch siedenden Verrdünnungsmittels umsetzt und dann gegebenenfalls mit Wasser sowie gegebenenfalls mit Säure behandelt,
und gegebenenfalls die nach den Varianten (α) und (β) entstehenden Verbindungen der Formel 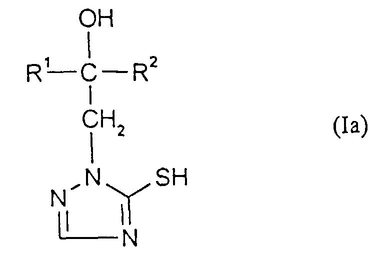 in welcher
R¹ und R² die oben angegebenen Bedeutungen haben,
mit Halogen-Verbindungen der Formel
R⁴-Hal (III)
in welcher
R⁴ für geradkettiges oder verzweigtes Alkyl mit 1 bis 6 Kohlenstoffatomen steht, wobei jeder dieser Reste einfach bis dreifach durch Fluor und/oder Chlor substituiert sein kann,
oder
für geradkettiges oder verzweigtes Alkenyl mit 2 bis 6 Kohlenstoffatomen steht wobei jeder dieser Reste einfach bis dreifach durch Fluor und/oder Chlor substituiert sein kann, oder für Phenylalkyl mit 1 bis 4 Kohlenstoffatomen im geradkettigen oder verzweigten Alkylteil steht, wobei jeder dieser Reste im Phenylteil einfach bis dreifach, gleichartig oder verschieden substituiert sein kann durch Halogen, Alkyl mit 1 bis 4 Kohlenstoffatomen und/oder Halogenalkyl mit 1 bis 4 Kohlenstoffatomen und 1 bis 5 Halogenatomen,
und
Hal für Chlor, Brom oder Iod steht,
in Gegenwart eines Säurebindemittels und in Gegenwart eines Verdünnungsmittels umsetzt und gegebenenfalls die dabei entstehenden Verbindungen der Formel 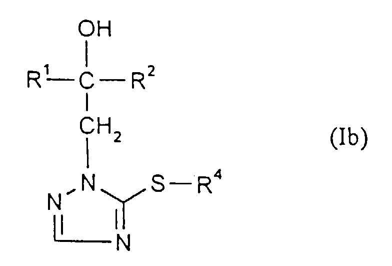 in welcher
R¹, R² und R⁴ die oben angegebenen Bedeutungen haben,
mit Oxidationsmitteln in Gegenwart eines Verdünnungsmittels umsetzt,
oder
b) Hydroxyethyl-triazole der Formel 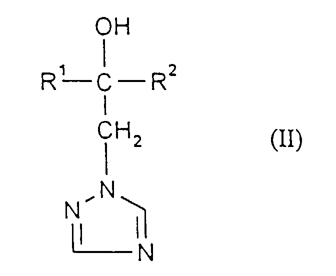 in welcher
R¹ und R² die oben angegebenen Bedeutungen haben,
nacheinander mit starken Basen und Diaryl-disulfiden der Formel
R⁵-S-S-R⁵ (IV)
in welcher
R⁵ für Phenyl steht, das einfach bis dreifach, gleichartig oder verschieden substituiert sein kann durch Halogen, Alkyl mit 1 bis 4 Kohlenstoffatomen und/oder Halogenalkyl mit 1 bis 4 Kohlenstoffatomen und 1 bis 5 Halogenatomen,
in Gegenwart eines Verdünnungsmittels umsetzt und gegebenenfalls die dabei entstehenden Verbindungen der Formel 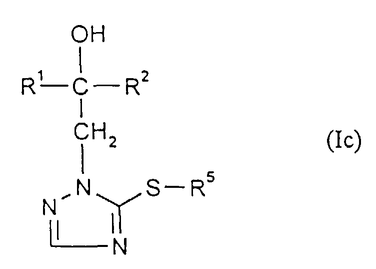 in welcher
R¹, R² und R⁵ die oben angegebenen Bedeutungen haben,
mit Oxidationsmitteln in Gegenwart eines Verdünnungsmittels umsetzt,
oder
c) Triazolyl-Derivate der Formel 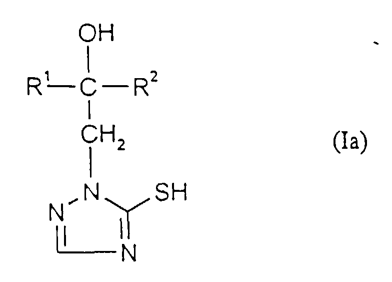 in welcher
R¹ und R² die oben angegebenen Bedeutungen haben,
mit Kaliumpermanganat in Gegenwart eines Verdünnungsmittels umsetzt,
und gegebenenfalls anschließend an die erhaltenen Verbindungen der Formel (I) eine Säure oder ein Metallsalz addiert.

5. Mikrobizide Mittel, gekennzeichnet durch einen Gehalt an mindestens einem Triazolyl-Derivat der Formel (I) gemäß Anspruch 1 bzw. an einem Säureadditions-Salz oder Metallsalz-Komplex eines Triazolyl-Derivates der Formel (I).

6. Verwendung von Triazolyl-Derivaten der Formel (I) gemäß Anspruch 1 sowie von deren Säureadditions-Salzen und Metallsalz-Komplexen als Mikrobizide im Pflanzenschutz und im Materialschutz.

7. Verfahren zur Bekämpfung von unerwünschten Mikroorganismen im Pflanzenschutz und im Materialschutz, dadurch gekennzeichnet, daß man Triazolyl-Derivate der Formel (I) gemäß Anspruch 1 bzw. deren Säureadditions-Salze oder Metallsalz-Komplexe auf die Mikroorganismen und/oder deren Lebensraum ausbringt.

8. Verfahren zur Herstellung von mikrobiziden Mitteln, dadurch gekennzeichnet, daß man Triazolyl-Derivate der Formel (I) gemäß Anspruch 1 bzw. deren Säureadditions-Salze oder Metallsalz-Komplexe mit Streckmitteln und/oder oberflächenaktiven Stoffen vermischt.

## Claims

1. Triazolyl derivatives of the formula 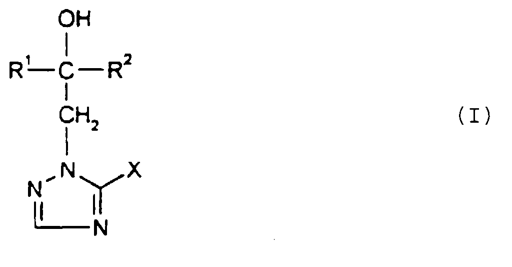 in which
R¹ represents straight-chain or branched alkyl having 1 to 6 carbon atoms, it being possible for these radicals to be monosubstituted to tetrasubstituted by identical or different substituents from the series consisting of halogen, alkoxy having 1 to 4 carbon atoms, alkoximino having 1 to 4 carbon atoms in the alkoxy moiety, and/or cycloalkyl having 3 to 7 carbon atoms,
or
R¹ represents cycloalkyl having 3 to 7 carbon atoms, it being possible for each of these radicals to be monosubstituted to trisubstituted by identical or different substituents from the series consisting of halogen, cyano and/or alkyl having 1 to 4 carbon atoms,
or
R¹ represents aralkyl having 6 to 10 carbon atoms in the aryl moiety and 1 to 4 carbon atoms in the straight-chain or branched alkyl moiety, it being possible for the aryl moiety in each case to be monosubstituted to trisubstituted by identical or different substituents from the series consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and/or cyano,
or
R¹ represents aroxyalkyl having 6 to 10 carbon atoms in the aryl moiety and 1 to 4 carbon atoms in the straight-chain or branched oxyalkyl moiety, it being possible for the aryl moiety in each case to be monosubstituted to trisubstituted by identical or different substituents from the series consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and/or cyano,
or
R¹ represents aryl having 6 to 10 carbon atoms, it being possible for each of these radicals to be monosubstituted to trisubstituted by identical or different substituents from the series consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and/or cyano,
or
R¹ represents an optionally benzo-fused five- or six-membered heteroaromatic radical having 1 to 3 heteroatoms, it being possible for each of these radicals to be monosubstituted to trisubstituted by identical or different substituents from the series consisting of halogen, alkyl having 1 to 4 carbon atoms, hydroxyalkyl having 1 to 4 carbon atoms, hydroxyalkinyl having 3 to 8 carbon atoms, alkoxy having 1 or 2 carbon atoms, alkylthio having 1 or 2 carbon atoms, halogenoalkyl, halogenoalkoxy and halogenoalkylthio, each of which has 1 or 2 carbon atoms and 1 to 5 identical or different halogen atoms, formyl, dialkoxymethyl having 1 or 2 carbon atoms in each alkoxy group, acyl having 2 to 4 carbon atoms, alkoxycarbonyl having 1 to 4 carbon atoms in the alkoxy moiety, alkoxyiminoalkyl having 1 to 4 carbon atoms in the alkoxy moiety and 1 to 3 carbon atoms in the alkyl moiety, nitro and/or cyano.
R² represents straight-chain or branched alkyl having 1 to 6 carbon atoms, it being possible for these radicals to be monosubstituted to tetrasubstituted by identical or different substituents from the series consisting of halogen, alkoxy having 1 to 4 carbon atoms, alkoximino having 1 to 4 carbon atoms in the alkoxy moiety and/or cycloalkyl having 3 to 7 carbon atoms,
or
R² represents cycloalkyl having 3 to 7 carbon atoms, it being possible for each of these radicals to be monosubstituted to trisubstituted by identical or different substituents from the series consisting of halogen, cyano and/or alkyl having 1 to 4 carbon atoms,
or
R² represents aralkyl having 6 to 10 carbon atoms in the aryl moiety and 1 to 4 carbon atoms in the straight-chain or branched alkyl moiety, it being possible for the aryl moiety in each case to be monosubstituted to trisubstituted by identical or different substituents from the series consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and/or cyano,
or
R² represents aroxyalkyl having 6 to 10 carbon atoms in the aryl moiety and 1 to 4 carbon atoms in the straight-chain or branched oxyalkyl moiety, it being possible for the aryl moiety in each case to be monosubstituted to trisubstituted by identical or different substituents from the series consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and/or cyano,
or
R² represents aryl having 6 to 10 carbon atoms, it being possible for each of these radicals to be monosubstituted to trisubstituted by identical or different substituents from the series consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and/or cyano,
or
R² represents an optionally benzo-fused five- or six-membered heteroaromatic radical having 1 to 3 heteroatoms, it being possible for each of these radicals to be monosubstituted to trisubstituted by identical or different substituents from the series consisting of halogen, alkyl having 1 to 4 carbon atoms, hydroxyalkyl having 1 to 4 carbon atoms, hydroxyalkinyl having 3 to 8 carbon atoms, alkoxy having 1 or 2 carbon atoms, alkylthio having 1 or 2 carbon atoms, halogenoalkyl, halogenoalkoxy and halogenoalkylthio, each of which has 1 or 2 carbon atoms and 1 to 5 identical or different halogen atoms, formyl, dialkoxymethyl having 1 or 2 carbon atoms in each alkoxy group, acyl having 2 to 4 carbon atoms, alkoxycarbonyl having 1 to 4 carbon atoms in the alkoxy moiety, alkoximinoalkyl having 1 to 4 carbon atoms in the alkoxy moiety and 1 to 3 carbon atoms in the alkyl moiety, nitro and/or cyano, and
X represents the groups -SH, -SR³, -SO-R³, -SO₂- R³ or -SO₃H, in which
R³ represents straight-chain or branched alkyl having 1 to 6 carbon atoms, it being possible for each of these radicals to be monosubstituted to trisubstituted by fluorine and/or chlorine,
or
R³ represents straight-chain or branched alkenyl having 2 to 6 carbon atoms, it being possible for each of these radicals to be monosubstituted to trisubstituted by fluorine and/or chlorine,
or
R³ represents phenylalkyl having 1 to 4 carbon atoms in the straight-chain or branched alkyl moiety, it being possible for each of these radicals to be monosubstituted to trisubstituted in the phenyl moiety by identical or different substituents from the series consisting of halogen, alkyl having 1 to 4 carbon atoms, and/or halogenoalkyl having 1 to 4 carbon atoms and 1 to 5 halogen atoms,
or
R³ represents phenyl which can be monosubstituted to trisubstituted by identical or different substituents from the series consisting of halogen, alkyl having 1 to 4 carbon atoms, and/or halogenoalkyl having 1 to 4 carbon atoms and 1 to 5 halogen atoms,
and their acid addition salts and metal salt complexes.

2. Triazolyl derivatives of the formula (I) according to Claim 1, in which
R¹ represents 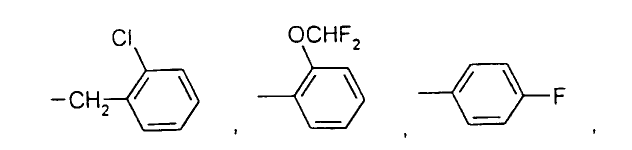 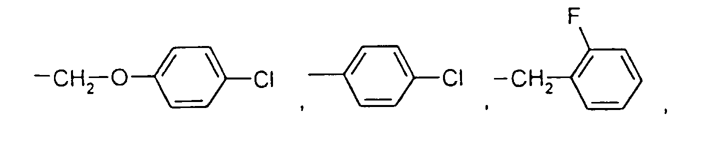 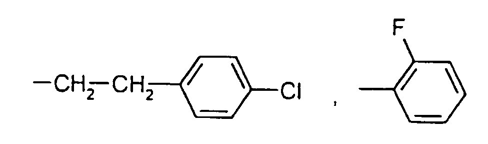 or 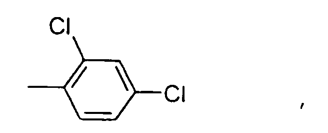
R² represents 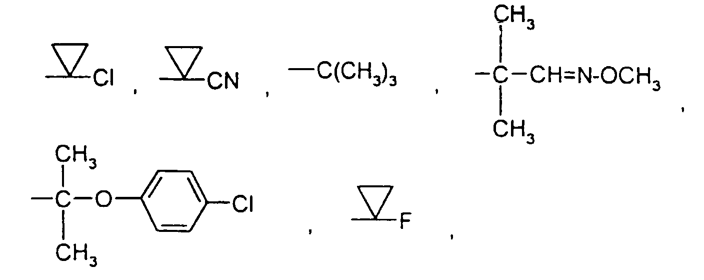 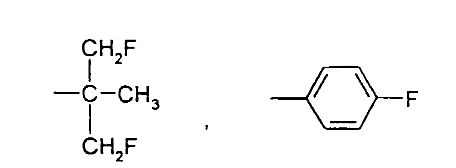 or -C₄H₉-n, and
X represents -SH, -SCH₃, -SO₂CH₃, -SO₃H or

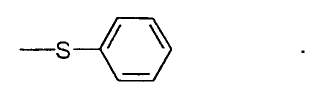

3. Triazolyl derivative according to Claim 1, characterized by the formula

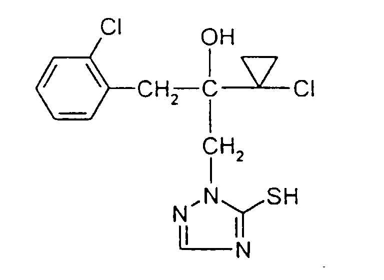

4. Process for the preparation of triazolyl derivatives of the formula (I) according to Claim 1
and of their acid addition salts and metal salt complexes, characterized in that
a) hydroxyethyl-triazoles of the formula 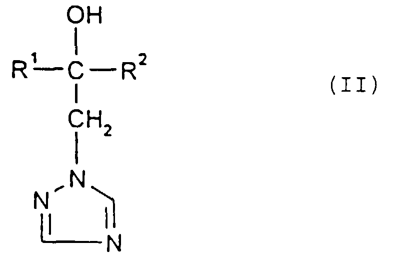 in which
R¹ and R² have the abovementioned meanings,
either
α) are reacted in succession with strong bases and sulphur in the presence of a diluent and then hydrolysed with water, optionally in the presence of an acid,
or
β) reacted with sulphur in the presence of a high-boiling diluent and then optionally treated with water and also optionally with acid,
and optionally the compounds which form in accordance with the variants (α) and (β), of the formula 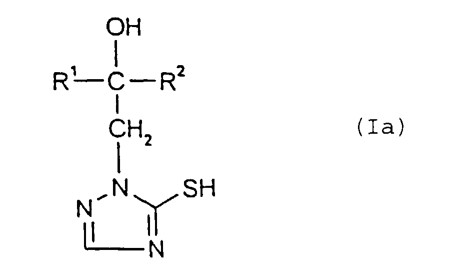 in which
R¹ and R² have the abovementioned meanings
are reacted with halogen compounds of the formula
R⁴-Hal (III)
in which
R⁴ represents straight-chain or branched alkyl having 1 to 6 carbon atoms, it being possible for each of these radicals to be substituted from one to three times by fluorine and/or chlorine,
or
represents straight-chain or branched alkenyl having 2 to 6 carbon atoms, it being possible for each of these radicals to be substituted from one to three times by fluorine and/or chlorine,
or
represents phenylalkyl having 1 to 4 carbon atoms in the straight-chain or branched alkyl moiety, it being possible for each of these radicals to be substituted in the phenyl moiety by from one to three identical or different substituents selected from the series consisting of halogen, alkyl having 1 to 4 carbon atoms and/or halogenoalkyl having 1 to 4 carbon atoms and 1 to 5 halogen atoms, and
Hal represents chlorine, bromine or iodine
in the presence of an acid-binding agent and in the presence of a diluent and, if appropriate, the resulting compounds of the formula 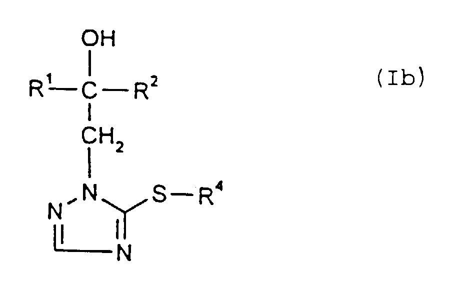
in which
R¹, R² and R⁴ have the abovementioned meanings
are reacted with oxidants in the presence of a diluent,
or
b) hydroxyethyl-triazoles of the formula 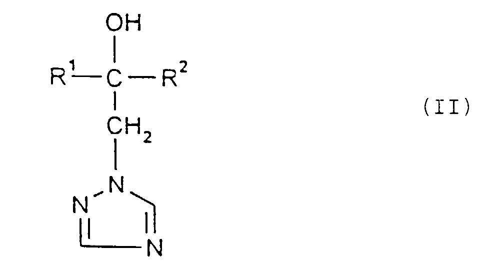 in which
R¹ and R² have the abovementioned meanings
are reacted in succession with strong bases and diaryl disulphides of the formula
R⁵-S-S-R⁵ (IV)
in which
R⁵ represents phenyl which can be substituted by from one to three identical or different substituents selected from the series consisting of halogen, alkyl having 1 to 4 carbon atoms and/or halogenoalkyl having 1 to 4 carbon atoms and 1 to 5 halogen atoms,
in the presence of a diluent and, if appropriate, the resulting compounds of the formula 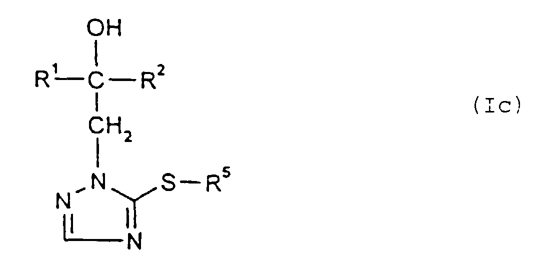 in which
R¹, R² and R⁵ have the abovementioned meanings
are reacted with oxidants in the presence of a diluent,
or
c) triazolyl derivatives of the formula 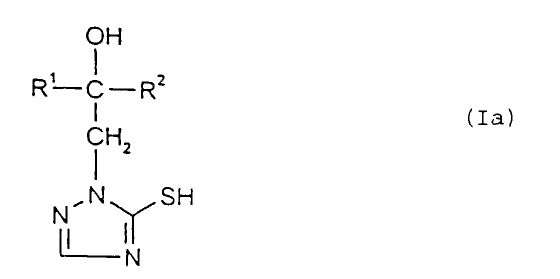 in which
R¹ and R² have the abovementioned meanings
are reacted with potassium permanganate in the presence of a diluent,
and, if appropriate, the resulting compounds of the formula (I) are then subjected to an addition reaction with an acid or a metal salt.

5. Microbicidal compositions, characterized in that they comprise at least one triazolyl derivative of the formula (I) according to Claim 1 or an acid addition salt or metal salt complex of a triazolyl derivative of the formula (I).

6. Use of triazolyl derivatives of the formula (I) according to Claim 1, and of their acid addition salts and metal salt complexes as microbicides in plant protection and protection of materials.

7. Method of combating undesirable microorganisms in plant protection and protection of materials, characterized in that triazolyl derivatives of the formula (I) according to Claim 1 or their acid addition salts or metal salt complexes are applied to the microorganisms and/or their environment.

8. Process for the preparation of microbicidal compositions, characterized in that triazolyl derivatives of the formula (I) according to Claim 1 or their acid addition salts or metal salt complexes are mixed with extenders and/or surface-active substances.

## Revendications

1. Dérivés de triazolyle de formule 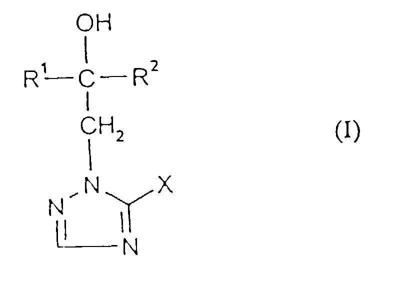 dans laquelle
R¹ est un reste alkyle linéaire ou ramifié ayant 1 à 6 atomes de carbone, les restes correspondants pouvant être substitués une à quatre fois identiques ou différentes par un halogène, un groupe alkoxy ayant 1 à 4 atomes de carbone, alkoximino ayant 1 à 4 atomes de carbone dans la partie alkoxy et/ou cycloalkyle ayant 3 à 7 atomes de carbone,
ou bien
R¹ est un reste cycloalkyle ayant 3 à 7 atomes de carbone, chacun des restes correspondants pouvant être substitué une à trois fois identiques ou différentes par un halogène, un radical cyano et/ou alkyle ayant 1 à 4 atomes de carbone,
ou bien
R¹ est un reste aralkyle ayant 6 à 10 atomes de carbone dans la partie aryle et 1 à 4 atomes de carbone dans la partie alkyle linéaire ou ramifiée, la partie aryle pouvant être substituée dans chaque cas une à trois fois identiques ou différentes par un halogène, un radical alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, cycloalkyle ayant 3 à 7 atomes de carbone, phényle, phénoxy, alkoxycarbonyle ayant 1 à 4 atomes de carbone dans la partie alkoxy, alkoximino-alkyle ayant 1 à 4 atomes de carbone dans la partie alkoxy et 1 à 4 atomes de carbone dans la partie alkyle, nitro et/ou cyano,
ou bien
R¹ est un reste aroxyalkyle ayant 6 à 10 atomes de carbone dans la partie aryle et 1 à 4 atomes de carbone dans la partie oxyalkyle linéaire ou ramifiée, la partie aryle pouvant être substituée dans chaque cas une à trois fois identiques ou différentes par un halogène, un radical alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, cycloalkyle ayant 3 à 7 atomes de carbone, phényle, phénoxy, alkoxycarbonyle ayant 1 à 4 atomes de carbone dans la partie alkoxy, alkoximino-alkyle ayant 1 à 4 atomes de carbone dans la partie alkoxy et 1 à 4 atomes de carbone dans la partie alkyle, nitro et/ou cyano,
ou bien
R¹ est un reste aryle ayant 6 à 10 atomes de carbone, chacun des restes correspondants pouvant être substitué une à trois fois identiques ou différentes par un halogène, un radical alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, cycloalkyle de 3 à 7 atomes de carbone, phényle, phénoxy, alkoxycarbonyle ayant 1 à 4 atomes de carbone dans la partie alkoxy, alkoximino-alkyle ayant 1 à 4 atomes de carbone dans la partie alkoxy et 1 à 4 atomes de carbone dans la partie alkyle, nitro et/ou cyano,
ou bien
R¹ représente un reste hétéroaromatique pentagonal ou hexagonal éventuellement condensé au benzène, ayant 1 à 3 hétéroatomes, chacun des restes correspondants pouvant être substitué une à trois fois identiques ou différentes par un halogène, un radical alkyle ayant 1 à 4 atomes de carbone, hydroxyalkyle ayant 1 à 4 atomes de carbone, hydroxyalcynyle ayant 3 à 8 atomes de carbone, alkoxy ayant 1 ou 2 atomes de carbone, alkylthio ayant 1 ou 2 atomes de carbone, halogénalkyle, halogénalkoxy et halogénalkylthio ayant 1 ou 2 atomes de carbone et 1 à 5 atomes d'halogènes identiques ou différents, formyle, dialkoxyméthyle ayant 1 ou 2 atomes de carbone dans chaque groupe alkoxy, acyle ayant 2 à 4 atomes de carbone, alkoxycarbonyle ayant 1 à 4 atomes de carbone dans la partie alkoxy, alkoximino-alkyle ayant 1 à 4 atomes de carbone dans la partie alkoxy et 1 à 3 atomes de carbone dans la partie alkyle, nitro et/ou cyano,
R² représente un reste alkyle linéaire ou ramifié ayant 1 à 6 atomes de carbone, les restes correspondants pouvant être substitués une à quatre fois identiques ou différentes par un halogène, un radical alkoxy ayant 1 à 4 atomes de carbone, alkoximino ayant 1 à 4 atomes de carbone dans la partie alkoxy et/ou cycloalkyle ayant 3 à 7 atomes de carbone,
ou bien
R² est un reste cycloalkyle ayant 3 à 7 atomes de carbone, chacun des restes correspondants pouvant être substitué une à trois fois identiques ou différentes par un halogène, un radical cyano et/ou alkyle ayant 1 à 4 atomes de carbone,
ou bien
R² est un reste aralkyle ayant 6 à 10 atomes de carbone dans la partie aryle et 1 à 4 atomes de carbone dans la partie alkyle linéaire ou ramifiée, la partie aryle pouvant être substituée dans chaque cas une à trois fois identiques ou différentes par un halogène, un radical alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, cycloalkyle ayant 3 à 7 atomes de carbone, phényle, phénoxy, alkoxycarbonyle ayant 1 à 4 atomes de carbone dans la partie alkoxy, alkoximino-alkyle ayant 1 à 4 atomes de carbone dans la partie alkoxy et 1 à 4 atomes de carbone dans la partie alkyle, nitro et/ou cyano,
ou bien
R² est un reste aroxyalkyle ayant 6 à 10 atomes de carbone dans la partie aryle et 1 à 4 atomes de carbone dans la partie oxyalkyle linéaire ou ramifiée, la partie aryle pouvant être substituée dans chaque cas une à trois fois identiques ou différentes par un halogène, un radical alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, cycloalkyle ayant 3 à 7 atomes de carbone, phényle, phénoxy, alkoxycarbonyle ayant 1 à 4 atomes de carbone dans la partie alkoxy, alkoximino-alkyle ayant 1 à 4 atomes de carbone dans la partie alkoxy et 1 à 4 atomes de carbone dans la partie alkyle, nitro et/ou cyano,
ou bien
R² représente un reste aryle de 6 à 10 atomes de carbone, chacun des restes correspondants pouvant être substitué une à trois fois identiques ou différentes par un halogène, un radical alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, cycloalkyle ayant 3 à 7 atomes de carbone, phényle, phénoxy, alkoxycarbonyle ayant 1 à 4 atomes de carbone dans la partie alkoxy, alkoximino-alkyle ayant 1 à 4 atomes de carbone dans la partie alkoxy et 1 à 4 atomes de carbone dans la partie alkyle, nitro et/ou cyano,
ou bien
R² représente un reste hétéroaromatique pentagonal ou hexagonal éventuellement condensé au benzène, ayant 1 à 3 hétéroatomes, chacun des restes correspondants pouvant être substitué une à trois fois identiques ou différentes par un halogène, un radical alkyle ayant 1 à 4 atomes de carbone, hydroxyalkyle ayant 1 à 4 atomes de carbone, hydroxyalcynyle ayant 3 à 8 atomes de carbone, alkoxy ayant 1 ou 2 atomes de carbone, alkylthio ayant 1 ou 2 atomes de carbone, halogénalkyle, halogénalkoxy et halogénalkylthio ayant chacun 1 ou 2 atomes de carbone et 1 à 5 atomes d'halogènes identiques ou différents, formyle, dialkoxyméthyle ayant 1 ou 2 atomes de carbone dans chaque groupe alkoxy, acyle ayant 2 à 4 atomes de carbone, alkoxycarbonyle ayant 1 à 4 atomes de carbone dans la partie alkoxy, alkoximino-alkyle ayant 1 à 4 atomes de carbone dans la partie alkoxy et 1 à 3 atomes de carbone dans la partie alkyle, nitro et/ou cyano, et
X représente l'un des groupements -SH, -SR³, -SO-R³, -SO₂-R³ ou -SO₃H, dans lesquels
R³ représente un reste alkyle linéaire ou ramifié ayant 1 à 6 atomes de carbone, chacun des restes correspondants pouvant être substitué une à trois fois par du fluor et/ou du chlore,
ou bien
R³ est un reste alcényle linéaire ou ramifié ayant 2 à 6 atomes de carbone, chacun des restes correspondants pouvant être substitué une à trois fois par du fluor et/ou du chlore,
ou bien
R³ est un reste phénylalkyle ayant 1 à 4 atomes de carbone dans la partie alkyle linéaire ou ramifiée, chacun des restes correspondants pouvant être substitué dans la partie phényle une à trois fois identiques ou différentes par un halogène, un radical alkyle ayant 1 à 4 atomes de carbone et/ou un radical halogénalkyle ayant 1 à 4 atomes de carbone et 1 à 5 atomes d'halogènes,
ou bien
R³ est un reste phényle qui peut être substitué une à trois fois identiques ou différentes par un halogène, un radical alkyle ayant 1 à 4 atomes de carbone et/ou un radical halogénalkyle ayant 1 à 4 atomes de carbone et 1 à 5 atomes d'halogènes,
ainsi que leurs sels d'addition d'acides et leurs complexes de sels métalliques.

2. Dérivés de triazolyle de formule (I) suivant la revendication 1, dans laquelle
R¹ représente 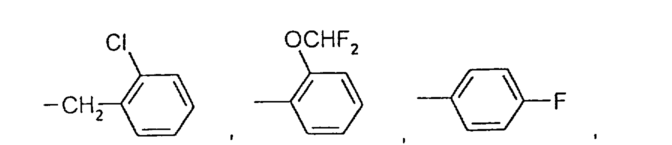 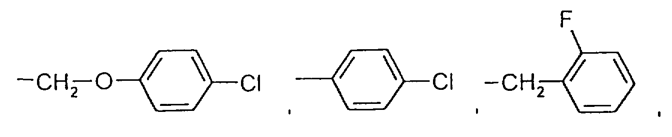 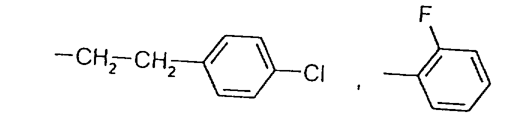 ou 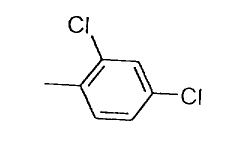
R² représente 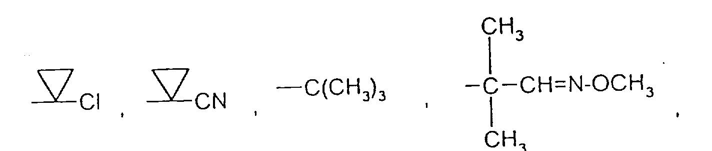 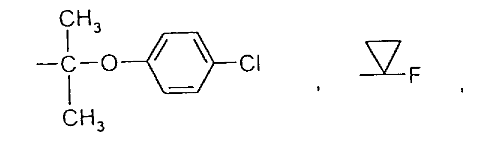 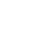 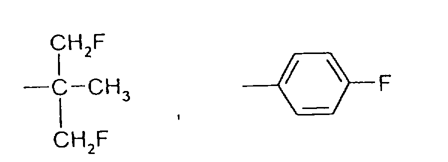 ou un groupe -C₄H₉-n et
X représente -SH-, -SCH₃, -SO₂CH₃, -SO₃H ou

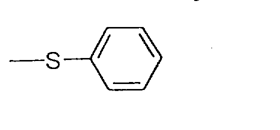

3. Dérivé de triazolyle suivant la revendication 1, caractérisé par la formule

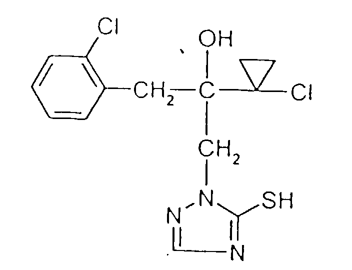

4. Procédé de production de dérivés de triazolyle de formule (I) suivant la revendication 1
ainsi que de leurs sels d'addition d'acides et de leurs complexes de sels métalliques, caractérisé en ce que
a) des hydroxyéthyl-triazoles de formule 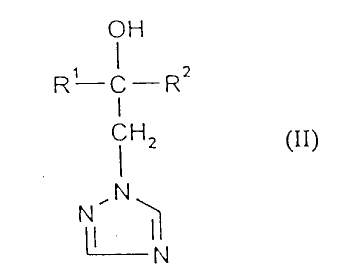 dans laquelle
R¹ et R² ont les définitions indiquées ci-dessus,
α) sont amenés à réagir successivement avec des bases fortes et du soufre en présence d'un diluant, puis le produit est hydrolysé avec de l'eau, éventuellement en présence d'un acide,
ou bien
β) sont amenés à réagir avec du soufre en présence d'un diluant de haut point d'ébullition, puis traités éventuellement avec de l'eau ainsi que le cas échéant avec un acide,
et les composés, produits selon les variantes (α) et (β), de formule 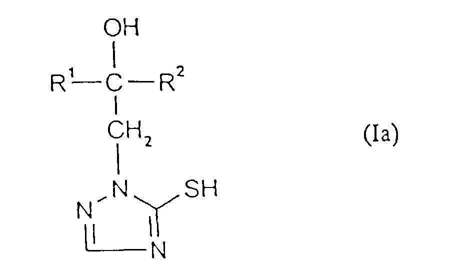 dans laquelle
R¹ et R² ont les définitions indiquées ci-dessus,
sont éventuellement amenés à réagir avec des composés halogénés de formule
R⁴-Hal (III)
dans laquelle
R⁴ est un reste alkyle linéaire ou ramifié ayant 1 à 6 atomes de carbone, chacun des restes correspondants pouvant être substitué une à trois fois par du fluor et/ou du chlore,
ou bien
un reste alcényle linéaire ou ramifié ayant 2 à 6 atomes de carbone, chacun des restes correspondants pouvant être substitué une à trois par du fluor et/ou du chlore,
ou bien
un reste phénylalkyle ayant 1 à 4 atomes de carbone dans la partie alkyle linéaire ou ramifiée, chacun des restes correspondants pouvant être substitué dans la partie phényle une à trois fois identiques ou différentes par un halogène, un radical alkyle ayant 1 à 4 atomes de carbone et/ou un radical halogénalkyle ayant 1 à 4 atomes de carbone et 1 à 5 atomes d'halogènes,
et
Hal représente le chlore, le brome ou l'iode,
en présence d'un accepteur d'acide et en présence d'un diluant et les composés alors produits, de formule 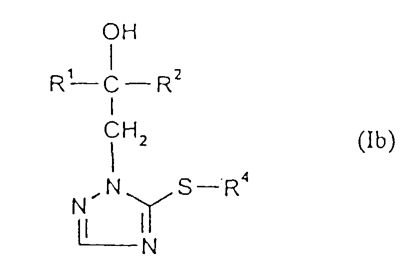 dans laquelle
R¹, R² et R⁴ ont les définitions données ci-dessus,
sont éventuellement amenés à réagir avec des oxydants en présence d'un diluant,
ou bien
b) des hydroxyéthyl-triazoles de formule 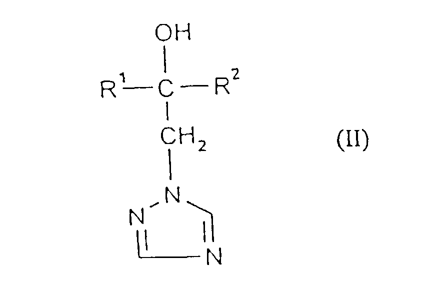 dans laquelle
R¹ et R² ont les définitions indiquées ci-dessus,
sont amenés à réagir successivement avec des bases fortes et des disulfures de diaryle de formule
R⁵-S-S-R⁵ (IV)
dans laquelle
R⁵ représente un reste phényle qui peut être substitué une à trois fois identiques ou différentes par un halogène, un radical alkyle ayant 1 à 4 atomes de carbone et/ou un radical halogénalkyle ayant 1 à 4 atomes de carbone et 1 à 5 atomes d'halogènes,
en présence d'un diluant et les composés alors produits, de formule 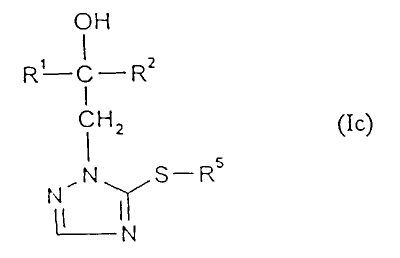 dans laquelle
R¹, R² et R⁵ ont les définitions indiquées ci-dessus,
sont éventuellement amenés à réagir avec des oxydants en présence d'un diluant,
ou bien
c) des dérivés de triazolyle de formule 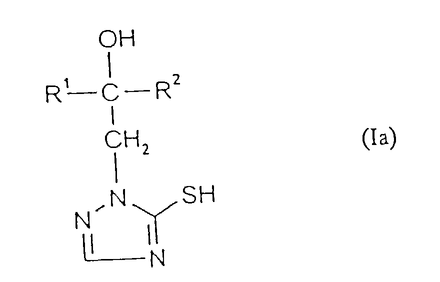 dans laquelle
R¹ et R² ont les définitions indiquées ci-dessus,
sont amenés à réagir avec du permanganate de potassium en présence d'un diluant,
puis, le cas échéant, un acide ou un sel métallique est additionné sur les composés de formule (I) ainsi obtenus.

5. Compositions microbicides, caractérisées par une teneur en au moins un dérivé de triazolyle de formule (I) suivant la revendication 1 ou en un sel d'addition d'acide ou un complexe de sel métallique d'un dérivé de triazolyle de formule (I).

6. Utilisation de dérivés de triazolyle de formule (I) suivant la revendication 1 ainsi que de leurs sels d'addition d'acides et de leurs complexes de sels métalliques comme microbicides dans la protection des plantes et dans la protection des matériaux.

7. Procédé pour combattre des micro-organismes indésirables dans la protection des plantes et dans la protection des matériaux, caractérisé en ce qu'on épand des dérivés de triazolyle de formule (I) suivant la revendication 1 ou leurs sels d'addition d'acides ou leurs complexes de sels métalliques sur les micro-organismes et/ou sur leur milieu.

8. Procédé de préparation de compositions microbicides, caractérisé en ce qu'on mélange des dérivés de triazolyle de formule (I) suivant la revendication 1 ou leurs sels d'addition d'acides ou leurs complexes de sels métalliques avec des diluants et/ou des agents tensio-actifs.
